(19) Europäisches Patentamt
European Patent Office
Office européen des brevets

(11) Publication number: **0 052 916**
**B1**

(12) **EUROPEAN PATENT SPECIFICATION**

(45) Date of publication of patent specification: **02.01.85**

(51) Int. Cl.[4]: **A 61 K 9/22**, A 61 L 15/03

(21) Application number: **81303310.7**

(22) Date of filing: **20.07.81**

(54) **Erodible polymer containing erosion rate modifier.**

(30) Priority: **10.11.80 US 205636**
**01.12.80 US 211510**

(43) Date of publication of application:
**02.06.82 Bulletin 82/22**

(45) Publication of the grant of the patent:
**02.01.85 Bulletin 85/01**

(84) Designated Contracting States:
**CH DE FR GB IT LI SE**

(56) References cited:
**US-A-3 553 117**
**US-A-4 131 648**
**US-A-4 159 322**
**US-A-4 180 646**

(73) Proprietor: **ALZA CORPORATION**
**950 Page Mill Road**
**Palo Alto, California 94304 (US)**

(72) Inventor: **Schmitt, Edward E.**
**848 Seale Avenue**
**Palo Alto California (US)**
Inventor: **Capozza, Richard C.**
**5102 E. Berneil Drive**
**Paradise Valley Arizona 85253 (US)**

(74) Representative: **Evans, David Charles et al**
**F.J. CLEVELAND & COMPANY 40-43, Chancery Lane**
**London, WC2A 1JQ (GB)**

**EP 0 052 916 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Field of the invention

This invention pertains to drug delivery devices. The devices comprise an erodible polymer, a drug, and an erosion rate modifier.

### Background of the invention

Medical science has long recognized the value of delivery devices comprising a drug and an erodible polymer. These devices are valuable because the erodible polymer blended with an effective amount of a drug, erodes at a rate controlled by the inherent chemistry of the polymer itself. Therefore, the device, delivers the drug at a controlled rate and in an effective amount, to a biological environment of use. A major advance towards satisfying this recognition was met by the novel poly(orthoesters) and poly(orthocarbonates) disclosed in United States Patent No. 4,180,646 issued to patentees Nam S. Choi and Jorge Heller. The patent discloses a unique class of polymers comprising a polymeric backbone having a repeating monomeric unit consisting of a hydrocarbon radical and a symmetrical dioxycarbon unit with a multiplicity of organic groups bonded thereto. These polymers reproducably erode in an aqueous or a biological fluid environment to innocuous products. The polymers are useful for delivering a beneficial agent at a controlled rate to the environment of use.

While the above polymers are outstanding and represent a pioneer advancement in the polymer and the delivery arts, and while they are useful for delivering numerous drugs to the environment of use, there is an occasional instance when it is desirable to modify the rate of erosion of the polymer to produce a more preferred dose rate of drug. For example, when the polymers are used in the form of implants in an animal environment of use, it may be therapeutically desirable to modify the rate of erosion of the polymer to achieve any one of a number of dose rates. The dose rates are adjusted by regulating the amount of drug released per unit time by the implant, which is controlled by the rate of polymer erosion. Controlling the release rate is also useful for extending the period of time the implant remains in the environment. It will be appreciated by those versed in the present arts, and in view of this presentation, that if an erosion rate modifier were made available for modifying the rate of erosion of the poly(orthoesters) and the poly(orthocarbonates), such a modifier would represent a valuable contribution and a useful improvement in the polymer and delivering arts.

This invention concerns erodible delivery devices. The devices comprise a body formed of a polymeric backbone having a repeating monomeric unit of a hydrocarbon radical and a symmetrical dioxycarbon group with a multiplicity of organic groups bonded thereto. The polymers have a controlled degree of hydrophobicity which correspondingly controls the degree of erosion in aqueous and biological fluids. The devices additionally contain an erosion rate modifier that cooperates with the polymer for regulating the rate of erosion of the polymer. A drug in the device is released at a controlled rate and in a therapeutically effective amount as the polymer bioerodes over time. The invention also concerns compositions comprising the polymers and the erosion rate modifiers.

United States Application 4131648 and United States Application 4180646 compositely provide a device for delivering a drug, the device comprising: (A) an erodible polymer of the formula:

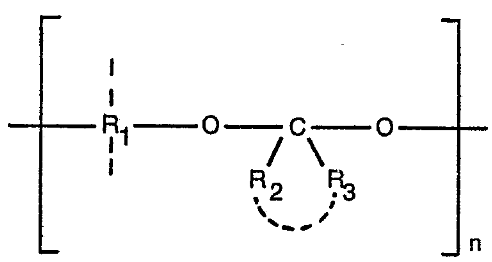

wherein *n* is greater than 10 and wherein (I) $R_1$ is selected from divalent, tribalent and tetravalent radicals consisting of alkylene of 1 to 10 carbons; alkenylene of 2 to 10 carbons; alkyleneoxy alkylene of 2 to 6 carbons; cycloalkylene of 3 to 7 carbons; cycloalkylene of 3 to 7 carbons substituted with an alkyl of 1 to 7 carbons, alkoxy of 1 to 7 carbons, an alkenyl of 2 to 7 carbons, and an alkylene of 1 to 10 carbons; cycloalkenylene of 4 to 7 carbons; optionally substituted with an alkyl of 1 to 7 carbons, an alkoxy of 1 to 7 carbons, alkenyl of 2 to 7 carbons, or an alkylene of 1 to 10 carbons; arylene of 6 to 12 carbons; optionally substituted with an alkyl of 1 to 7 carbons, and alkylene of 1 to 10 carbons; or

wherein (II) $R_2$ and $R_3$ are selected from alkyl of 1 to 7 carbons, alkenyl of 2 to 7 carbons, aryl of 6 to 12 carbons, an oxygen atom covalently bonded to the dioxycarbon atom, and when an oxygen atom $R_2$ and $R_3$ additionally include an alkyloxy of 1 to 7 carbons; alkenyloxy of 2 to 7 carbons; and aryloxy of 6 to 12 carbons; and when only one of $R_2$ and $R_3$ is selected from said member the other $R_2$ and $R_3$ is selected from alkyl of 1 to 7 carbons; alkenyl of 2 to 7 carbons; and aryl of 6 to 12 carbons; or

wherein (III) $R_2$ and $R_3$ are covalently bonded to each other and to the same dioxycarbon atom, with at least one of $R_2$ and $R_3$ being a ring oxygen atom forming a heterocyclic ring of 5 to 8 carbon and

oxygen atoms when $R_2$ and $R_3$ are selected from alkylene of 2 to 6 carbons; alkenylene of 2 to 6 carbons; alkylenedioxy of 2 to 5 carbons; alkenylenedieoxy of 2 to 5 carbons; oxa; and a heterocyclic ring of 5 to 8 carbon and oxygen atoms substituted with an alkyl of 1 to 7 carbons, an alkyloxy of 1 to 7 carbons; an alkenyl of 2 to 7 carbons, and an alkenyloxy of 2 to 7 carbons; or

wherein (IV) $R_2$ and $R_3$ are intramolecularly covalently bonded to each other and to the same dioxycarbon atom with at least one of $R_2$ and $R_3$ being a ring oxygen atom forming a fused polycyclic ring of 8 to 12 carbon and oxygen atoms when $R_2$ and $R_3$ are a member selected from

aralkylene of 8 to 12 carbons; aralkenylene of 8 to 12 carbons, aryloxy of 8 to 12 carbons; aralkyleneoxy of 8 to 12 carbons; aralkenyleneoxy of 8 to 12 carbons, aralkylenedioxy of 8 to 12 carbons; aralkenylenedioxy of 8 to 12 carbons; oxa; and a fused polycyclic ring of 8 to 12 carbon and oxygen atoms substituted with an alkyl of 1 to 7 carbons, an alkyloxy of 1 to 7 carbons, an alkenyl of 2 to 7 carbons and an alkenyloxy of 2 to 7 carbons; or

wherein (V) $R_2$ and $R_3$ are intramolecularly covalently bonded to each other and to the same dioxycarbon atom to form a 5 to 6 member carbocyclic ring; (B) a beneficial drug in the device;

The present invention provides the improvement characterized by the inclusion of an erosion rate modifier in the device which acts to either increase of decrease the rate of drug release by controlling the pH at the polymer-modifier-fluid-drug interface in use to a desired value different from the pH of the environment, to provide a desired erosion rate for the polymer selected, and correspondingly control the release rate of the drug from the device.

In the drawings, which are not drawn to scale, but are set forth to illustrate various embodiments of the invention, the figures are as follows:

Figure 1 is a view of a matrix made from the bioerodible polymer containing drug and release rate erosion modifier;

Figure 2 is a cross-sectional view through 2—2 of Figure 1 for illustrating the matrix having the drug and the modifier distributed throughout the matrix;

Figure 3 is a perspective view of a drug releasing assembly manufactured in the form of a strip as provided by the invention;

Figure 4 is a side, sectional view of the internal structure of the drug releasing assembly manufactured in the form of a strip as shown in Figure 3; and,

Figure 5 is a side view of a rod-like surgical implant provided by the invention for releasing drug in animal tissues.

Figures 6 to 16 indicate results obtained by using the modifiers of the invention.

Detailed description of the invention

Turning now to the drawings, which are examples of delivery devices, one embodiment of a delivery device is indicated in Figure 1 by the numeral 10. In Figure 1, device 10 comprises a matrix 12 made of a bioerodible poly(orthoester), or a bioerodible poly(orthocarbonate), which device 10 can be used for the administration of drug, not seen in Figure 1, to any drug receptor site. Device 10 delivers the drug by surface 11 bioeroding in a fluid environment at a controlled and continuous rate with the drug delivered at a corresponding controlled and continuous rate over a prolonged period of time.

Figure 2 is a cross-section through 2—2 of Figure 1 for illustrating the internal structures of matrix 12 which defines device 10. Matrix 12 is formed of a release rate bioerodible poly(orthoester) or poly(orthocarbonate) 14, and it houses drug 15, represented by dots, and an erosion rate modifier 16, represented by wavy lines. Erosion rate modifier 16, is a biologically acceptable material that cooperates with bioerodible polymer 14, for increasing the rate of erosion, or decreasing the rate of erosion of the polymer over time. A discussion of erosion rate modifiers is presented later in the specification.

Figure 3 illustrates another delivery device 10. Device 10, as seen in Figure 3, is manufactured in the form of a strip 17. Strip 17 can be applied to the animal body by adhesive or mechanical mean, and it comprises a main strip 18 formed of a flexible plastic such as polyurethane, polyvinyl chloride, natural rubber or aluminum foil having a central portion 19 with a pair of opposite side panels 20 and 21. The central portion has a raised section that forms a chamber 23 for housing a bioerodible polymer, which may include a fabric such as gauze or the like, or a thickening agent such as Cab-o-sil®, not shown in Figure 3. Side panels 20 and 21 serve as an attachment means for fixing strip 17 to a drug receptor surface, such as skin.

Figure 4 illustrates the structure of strip 17 taken through 4—4 of Figure 3. In Figure 4, a combination of the foregoing is shown, with chamber 23 operating as a medicament supplying depot. Chamber 23 comprises bioerodible polymer 14, represented by sectional lines, which polymer houses drug 15 and erosion rate modifier 16. Polymer 14 with or without gauze or thickening agents may possess adhesive-like properties for holding chamber 23 in tight contact with the skin, or optionally panels 20 and 21 can carry a thin layer of adhesive 24, such as silicone medical adhesive, a skin adhesive prepared by emulsion polymerization of dimethylaminoethyl methacrylate with an alkyl acrylate, or the like for holding strip 17 on the skin.

Figure 5 depicts a side view of a rod-shaped delivery device 10 particularly adapted for use as an implant. Implant 10 is manufactured for administering a drug within an animal body at a controlled and

continuous rate over a prolonged period of time. The depot implant is made from the bioerodible poly(orthoester-orthocarbonates) discussed herein, and it houses drug and an erosion rate modifier, as previously described in Figure 4. The implant can be introduced into the animal by a surgical operation, or the implant can be made as an injectable implant for insertion by trochar injection and retention in a muscle. The implant is sized, shaped and structured for placement subcutaneously, intradermally, or in a muscle of an animal wherein it bioerodes and releases drug over a prolonged period of time. Because the implant is bioerodible it requires only introduction into the animal, and because it erodes into innocuous products that are eliminated by the animal, and it never needs to be retrieved when all the drug is deployed.

While Figures 1 through 5 are illustrative of various delivery devices it is to be understood these devices can take a wide variety of shapes, sizes and forms that are structured and adapted for delivering drugs to the eye, vagina, uterus or anus, etc.

The devices of the invention are made from release rate controlling bioerodible poly(orthoesters) and poly(othocarbonates), erosion rate modifiers and drugs, wherein the polymers are represented by the following formula:

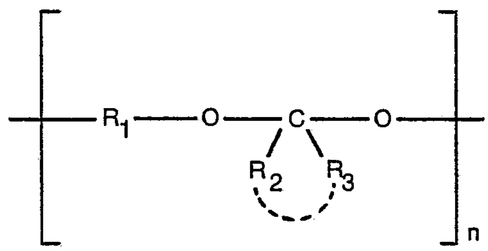

wherein (I) $R_1$ is a member selected from the group of divalent, trivalent and tetravalent radicals consisting of alkylene of 1 to 10 carbons; alkenylene of 2 to 10 carbons; alkyleneoxy of 2 to 6 carbons; cycloalkylene of 3 to 7 carbons; cycloalkylene of 3 to 7 carbons substituted with an alkyl of 1 to 7 carbons, alkoxy of 1 to 7 carbons, and alkenyl of 2 to 7 carbons, and an alkylene of 1 to 10 carbons; cycloalkenylene of 4 to 7 carbons; cycloalkenylene of 4 to 7 carbons substituted with an alkyl of 1 to 7 carbons, and alkoxy of 1 to 7 carbons, alkenyl of 2 to 7 carbons, and an alkylene of 1 to 10 carbons; arylene of 6 to 12 carbons; arylene of 6 to 12 carbons substituted with an alkyl of 1 to 7 carbons, alkyloxy of 1 to 7 carbons, an alkenyl of 2 to 7 carbons, and an alkylene of 1 to 10 carbons; wherein (II) $R_2$ and $R_3$ are alkyl of 1 to 7 carbons, alkenyl of 2 to 7 carbons, aryl of 6 to 12 carbons, an oxygen atom covalently bonded to the dioxycarbon atom, and when an oxygen atom $R_2$ and $R_3$ are a member independently selected from the group consisting of alkyloxy of 1 to 7 carbons; alkenyloxy of 2 to 7 carbons; and aryloxy of 6 to 12 carbons; and when only one of $R_2$ and $R_3$ is selected from said member the other $R_2$ and $R_3$ is selected from the group consisting of alkyl of 1 to 7 carbons; alkenyl of 2 to 7 carbons; and aryl of 6 to 12 carbons; wherein (III) $R_2$ and $R_3$ are intramolecularly covalently bonded to each other and to the same dioxycarbon atom, with at least one of $R_2$ and $R_3$ a ring oxygen atom, forming a heterocyclic ring of 5 to 8 carbon and oxygen atoms when $R_2$ and $R_3$ are selected from the group consisting of alkylene of 2 to 6 carbons; alkenylene of 2 to 6 carbons; alkyleneoxy of 2 to 6 carbons; alkenyleneoxy of 2 to 6 carbons; alkyldenedioxy of 2 to 5 carbons; alkenylenedioxy of 2 to 5 carbons; oxa; and a heterocyclic ring of 5 to 8 carbon and oxygen atoms substituted with an alkyl of 1 to 7 carbons, an alkyloxy of 1 to 7 carbons, an alkenyl of 2 to 7 carbons, and an alkenyloxy of 2 to 7 carbons; wherein (IV) $R_2$ and $R_3$ are intramolecularly covalently bonded to each other and to the same dioxycarbon atom, with at least one of $R_2$ and $R_3$ is a ring oxygen atom forming a fused polycyclic ring of 8 to 12 carbon and oxygen atoms when $R_2$ and $R_3$ are a member selected from the group consisting an aralkylene of 8 to 12 carbons; aralkyenylene of 8 to 12 carbons; aryloxy of 8 to 12 carbons; aralkyleneoxy of 8 to 12 carbons; aralkenyleneoxy of 8 to 12 carbons; aralkylenedioxy of 8 to 12 carbons; aralkenylenedioxy of 8 to 12 carbons; oxa; and a fuzed polycyclic ring of 8 to 12 carbon and oxygen atoms substituted with an alkyl of 1 to 7 carbons, an alkyloxy of 1 to 7 carbons; an alkenyl of 2 to 7 carbons; and an alkenyloxy of 2 to 7 carbons; wherein (V) $R_2$ and $R_3$ are intramolecularly covalently bonded to each other and to the same dioxycarbon atom to form a 5 to 6 member carbocyclic ring; and n is 10 to $10^7$.

The term alkylene as used in this specification and the accompanying claims denotes a straight or branched divalent, trivalent or tetravalent alkylene radical such as 1,2-ethylene; 1,3-propylene; 1,2-propylene; 1,4-butylene; 1,5-pentylene; 1,6-hexylene; 1,2,5-hexylene; 1,3,6-hexylene; 1,7-heptylene; 2-methyl-1,7-heptylene; 1,8-octylene; 1,10-decylene; and the like.

The term alkenylene denotes an unsaturated, straight or branched chain such as 1,4-but-2-enylene; 1,6-hex-3-enylene; 1,7-hept-3-enylene; 1,8-oct-3-enylene; 1,9-non-3-enylene; 4-propyl-(1,6-hex-3-enylene); 5-methoxy-(1,6-hex-3-enylene); 2-propenyl-(1,6-hex-3-enylene); and the like.

The term cycloalkylene includes monocyclic cycloalkylene radicals such as cyclopropylene; cyclobutylene; cyclopentylene; cyclophexylene; and cycloheptylene. Similarly, the phrase cycloalkylene substituted with an alkyl, and alkyloxy, an alkenyl, or alkylene includes substituted cycloalkylenes such as

2-methyl-1,3-cyclopropylene; 2-methyl-1,4-cyclopentylene; 2-methyl-1,6-cyclohexylene; 1-methylene-cyclohexylene; 1,6-dimethylene-cyclohexylene; 2-ethoxy-2,3-cyclopropylene; 5-butoxy-1,4-cyclopentylene; 2-methoxy-1,4-cyclohexylene; 2-propenyl-1,5-cyclopentylene; 2-isobutenyl-1,6-cyclohexylene; and the like.

Exemplary cycloalkenylene and cycloalkenylene substituted with an alkyl, an alkoxy, an alkenyl, or an alkylene include monocyclic cycloalkenylenes such as 1,4-cyclopent-2-enylene; 1,5-cyclopent-3-enylene; 1,6-cyclohex-2-enylene; 1,6-dimethylene-cyclohex-2-enylene; 5-methyl-(1,4-cyclo-pent-2-enylene); 6-ethyl-(1,4-cyclohex-2-enylene); 6-ethoxy-(1,5-cyclohex-2-enylene); 2-propyl-(1,5-cyclohex-3-enylene); 2-methoxy-(1,4-cyclohex-2-enylene); 2-methoxy-(1,4-cyclohept-2-enylene); 1-methylene-(1,6-cyclohex-2-enylene); and the like.

The expressions arylene and arylene substituted with an alkyl, an alkenyl, an alkoxy or an alkylene include the benzoid groups such as phenylene; phenylalkylene; phenylalkenylene; 1,4-phenyl-dimethylene; 1,4-phenyldiethylene; 2-ethyl-1,4-phenyldimethylene; 2-methyl-1,4-phenyl-dimethylene; 2-methoxy-1,4-phenylene; 2-propyl-1,4-phenylene; 2-propenyl-1,4-phenydimethylene; naphthalene; and the like.

The term alkyl embraces straight and branched chain alkyl radicals such as methyl; ethyl; n-propyl; n-butyl; n-amyl; n-hexyl; n-heptyl; and the various positional isomers such as isopropyl; t-butyl; sec-butyl; isoamyl; isohexyl; t-heptyl; and the like.

The term alkenyl embraces straight and branched chain lower alkenyl groups such as 1-propenyl; 2-propenyl or allyl; 1-butenyl; 2-butenyl; 1-pentenyl; 2-ethenyl; and the corresponding positional isomers such as 1-isobutenyl; 2-isobutenyl; 2-sec-butenyl; 2-methyl-1-butenyl; 2-methyl-2-pentenyl; 2,3-dimethyl-3-hexenyl; and the like.

The terms alkoxy or alkyloxy include the straight and branched chain lower groups, and the positional isomers thereof, such as methoxy; ethoxy; propoxy; butoxy; n-pentoxy; n-hexoxy; isopropoxy; 2-butoxy; isobutoxy; 3-pentoxy; and the like.

The term alkenyloxy embraces straight and branched chain lower alkenyloxy groups and the positional isomers thereof, such as ethenoxy; propenoxy; butenoxy; pentenoxy; hexenoxy; isopropenoxy; isobutenoxy; sec-butenoxy; 2-methyl-1-butenoxy; 2-methyl-2-butenoxy; 2,3-dimethyl-3-butenoxy; and the like.

The term alkyleneoxy comprehends straight and branched chain alkyleneoxy radicals such as 1,3-propyleneoxy; 1,4-butyleneoxy; 1,5-pentyleneoxy; 1,6-hexyleneoxy; 2,2-dimethyl-1,4-butyleneoxy; and the like. Similarly, the term alkenyleneoxy comprehends radicals such as prop-1-enyleneoxy; 1,4-but-1-enyleneoxy; 1,4-but-2-enyleneoxy; 1,5-pent-1-enyleneoxy; 1,6-hex-1-enyleneoxy; and the like.

The expressions alkylenedioxy and alkenylenedioxy include straight and branched chain radicals such as 1,3-propylenedioxy; 1,4-butylenedioxy; 1,5-pentylenedioxy; 1,6-hexylenedioxy; and 1,7-heptylenedioxy; 1,3-prop-1-enylenedioxy; 1,4-but-1-enylenedioxy; 1,4-but-2-enylenedioxy; 1,5-pent-1-enylenedioxy; 1,6-hex-1-enylenedioxy; and the like.

The terms aryloxy, aralkyleneoxy, aralkenyleneoxy; aralkylenedioxy, and aralkenylenedioxy indicate an aryl such as phenyl, aryloxy a phenyloxy, with the alkyleneoxy, alkenyleoneoxy, alkylenedioxy and alkenylenedioxy area as defined above.

The phrase, heterocyclic ring of 5 to 8 carbon and oxygen atoms formed when $R_2$ and $R_3$ are taken together, are represented by heterocyclic rings such as dioxolanyl dioxanyl; dioxepanyl; dioxocanyl; dioxonanyl; tetrahydrofuranyl; furyl; dihydrofuranyl; pyranyl; ocanyl; and oxepanyl.

The phrase, fused polycyclic ring with at least one or two ring forming oxygen atoms define a ring structure in which a heterocyclic and an aryl ring have two carbon atoms in common, are for example, a member selected from the group consisting of benzfuryl; benzpyranyl; 4,5-benz-1,3-dioxepanyl; 5,6-benz-1,3-dioxepanyl; 4,5-benz-1,3-dioxolanyl; 4,5-benz-1,3-dioxyolanyl; 4,5-benz-1,3-dioxocanyl; 5,6-benz-1,3-dioxocanyl; 6,7-benz-1,3-dioxocanyl; 6,7-benz-1,3-dioxocanyl; and benz-1,3-dioxanyl.

The phrase carbocyclic ring of 5 to 6 carbons formed when $R_2$ and $R_3$ are taken together are represented by rings such as cyclopentylene; cyclohexylene; cyclopentenylene; cyclopentadienylene; and phenylene.

The poly(orthoesters), and the poly(orthocarbonates) described herein are known to the prior art in United States Patent Nos. 4,093,709; 4,131,648; 4,138,344; and 4,180,646. These patents are issued to patentees Nam S. Choi and Jorge Heller, and they are assigned to the ALZA Corporation of Palo Alto, California.

The phrase, erosion rate modifier denotes a material that assists in controlling the rate of erosion of the erodible poly(orthoesters), or the rate of erosion of the poly(orthocarbonates). The modifiers can decrease the rate of erosion of the polymers and correspondingly increase the period of time the delivery device is in the environment of use releasing drug, or the modifier can increase the rate of erosion and correspondingly decrease the period of time the drug delivery device is in the environment of use releasing drug.

The erosion rate modifiers are therefore defined as those that increase the life of devices made from the erodible polymers are materials that mix with the polymers and drugs, and when the device is in a fluid environment of use, the modifier aids in producing an environment exhibiting a pH of at least 7 or greater at the polymer-modifier fluid-drug interface; the erosion rate modifiers that decrease or

lessen the life of devices made from the erodible polymers are materials that mix with the polymers and drugs, and when the device is in a fluid environment of use, the modifier aids in producing an environment exhibiting a pH of 7 or less at the polymer-modifier-fluid-drug interface.

Erosion rate modifiers that decrease the rate of erosion or prolong the life of the devices include a member selected from the group consisting of metal oxides such as binary compounds of oxygen; salts of electronegative nonmetal oxides such as mono, and dihydrogen phosphates, carbonates, sulfates, and the like; hydrides such as compounds of hydrogen with an electro-positive element such as lithium, sodium, potassium, magnesium, aluminum, alkali earths, and the like; metals and hydroxides of alkali earth metals; and tertiary compounds of nitrogen such as amines, substituted amines and polyamines. Erosion rate modifiers that increase the rate of erosion or shorten the life of the devices include a member selected from the group consisting essentially of organic acids, inorganic acids, Lewis acids, monobasic acid salts, polybasic acid salts, and hydroxides of nonmetals. The erosion rate modifiers also include surfactants. Particularly anionic surfactants that have a rate accelerating effect on the rate of erosion, and cationic surfactants that retard the rate of erosion. The amount of modifier used for the present purpose generally is about 0.001% to 40% by weight based on 100% by weight of the device. Additionally, the device can contain a multiplicity of erosion rate modifiers as used for the intended purpose.

Representative salts of electronegative nonmetal oxides include mono and dihydrogen phosphates, carbonates, sulfates and the like. Representative hydrides include compounds of hydrogen with electro-positive elements such as lithium, sodium, potassium, magnesium, aluminum, and alkali earth.

Representative metal oxides include aluminum oxide, calcium oxide, lithium oxide, magnesium oxide, potassium oxide, and sodium oxide.

Representative salts of nonmetal oxides include calcium carbonate, lithium carbonate, magnesium carbonate, sodium acid carbonate, potassium carbonate and sodium carbonate; calcium phosphate, lithium phosphate, disodihydrogen phosphate; magnesium phosphate, magnesium phosphate, manganese phosphate, potassium dihydrogen phosphate, potassium phosphate, sodium hydrogen phosphate, sodium phosphate; calcium sulfite, lithium, sulfite, magnesium sulfite, potassium sulfite and sodium sulfite; and lithium sulfate, potassium sulfate, sodium sulfate, and the like.

Representative metals include a member selected from the group consisting essentially of aluminum, calcium, lithium, sodium, magnesium, and potassium.

Representative hydride include a member selected from the group consisting of aluminum hydride, calcium hydride, lithium hydride, magnesium hydride, potassium hydride, and sodium hydride.

Representative hydroxides useful for the present purposes include compounds selected from the group consisting of aluminum hydroxide, barium hydroxide, calcium hydroxide, lithium hydroxide, magnesium hydroxide, potassium hydroxide, and sodium hydroxide.

Representative amine compounds include trimethylamine, triethylamine, tridecylamine, hydroxylamine, polylysine, propylamine, polypropylamine, ethylenediamine, and 2-hydroxy-1-aminopropane.

Representative organic and inorganic acids include amino acids, amygladic, adipic, boric, citric, fumaric, itaconic, lactic, glycine, malic, maleic, mesaconic, oxalic, phthalic, phosphoric, succinic, sulfamic, and tartaric. Lewis acids include aluminum chloride, stannic chloride, and mixtures thereof.

Representative of monobasic or polybasic acids, and acid salts include potassium tetraoxalate, potassium hydrogen tartrate, potassium hydrogen phthalate, sodium tetraoxalate, sodium hydrogen tartrate, sodium hydrogen phthalate, a mixture of sodium tetraoxalate and sodium acid carbonate, potassium dihydrogen citrate, sodium dihydrogen citrate, citric acid and sodium acetate, glycine and citric acid, and sodium p-toluenesulfonate, and p-toluenesulfonic acid.

The inorganic and organic compounds used for the novel purpose of the invention are disclosed in *Handbook of Chemistry and Physics*, 57th Edition, pags D-133 to D-134, 1976, published by CRC Press, Cleveland, Ohio; *Encyclopedia of Chemical Technology*, Vol. 11, pages 389 to 390, 1971, published by Wiley-Interscience, New York; *Lange's Handbook of Chemistry*, pages 5—73 to 5—80, 1979, published by McGraw-Hill, New York; and in *The Chemist's Companion*, by Gordon et al., pages 71 to 72, published by Wiley Interscience, New York.

Typical anionic surfactants generically include sulfated esters sulfated amides, sulfated alcohols, sulfated ethers, sulfated carboxylic acids, sulfonated aromatic hydrocarbons, sulfonated aliphatic hydrocarbons, sulfonated esters, sulfonated amids, sulfonated ethers, acylated amino acids, acylated polypeptides, and the like. Examples of specific surfactants include sodium oleate, sodium lauryl sulfate, sodium cetyl sulfate, sodium stearly sulfate, sodium alkylbenzene sulfonate, dialkyl sulfosuccinate; dioctylester of sodium sulphosuccunic acid; sodium salt of alkylated aryl polyether sulfate; and the like.

Typical cationic surfactants generically include primary alkylammonium salts, secondary alkylammonium salts, tertiary alkylammonium salts, quarternary alkylammonium salts, acylated polyamides, salts of heterocylic amines, benzyl ammonium salts, and the like. Examples of specific cationic surfactants include lauryldimethyl benzylammonium chloride; diisobutylphenoxyethoxyethyl-dimethyl benzylammonium chloride; alkyldimethylbenzylammonium chloride; laurylisoquinolinium bromide; cetylethyldimethylammonium chloride; stearyldimethyl benzylammonium chloride;

ethanolated alkyl guanidine amine complex; and the like. The amount of surfactant used for the present purpose is generally about 0.001% to 5% by weight based in the total weight of the device. The surfactants are known in Surface Active Agents, by *Schwartz and Perry*, 1949, Interscience Publishers, New York; *Harry's Cosmeticology* edited by Wilkinson, 1973, Chemical Publishing Co., Inc. and, *Systemic Analysis of Surface Active Agents*, Rosen and Goldsmith, 1972, Wiley-Interscience, new York.

The term drug as used herein denotes both locally and systemically acting drugs. Generically, the drugs include a member selected from the group consisting of analgesic, antiarthritic, antibiotic, antidepressant, antidiabetic, antihistaminic, antineoplastic, antipyretic, anticholinergic, antiinflammatory, anesthetic, antiviral, antiulcer, bronchial dilator, cardiovascular, contraceptive, decongestants, hypoglycemics, hormone, hypnotic, hematicinic, muscle relaxant, parasympatholytic, parasympathomimetic, ophthalmic, sedative, sympathomimetic, tranquilizer, uterine, vaginal, vasodilator, and the like. The drugs and their dosage unit amounts for humans are well known in *Pharmacology in Medicine*, by Drill and edited by DiPalma, 1965, published by McGraw-Hill, New York; in *Pharmacological Basis of Therapeutics*, by Goodman and Gilman, 4th Edition, 1970, published by MacMillian Co., and in United States Patent No. 3,977,404. The amount of drug present in a device provided by the invention will be about 0.5% to 50% by weight, with a more presently preferred amount of about 10% to 40% by weight.

Examples of drug that are administered by the devices over a prolonged period of time are hormones selected from the group consisting of cortical, androgenic, estrogenic and progestational hormones. Representative hormones include aldosterone, cortisone, hydrocortisone, prednisone, prednisolone, triamcinolone, dexamethasone, prednisolone, and the like.

Representative of estrogenic hormones include a member selected from the group consisting essentially of estradiol, β-estradiol, 17β-estradiol-17(disodium phosphate), quinestrol, and the like.

Representative of progestational hormones include natural progestins such as progesterone and preganediol, and synthetic progestins such as a member selected from the group consisting of chlormadinone acetate, hydroxyprogesterone, medrogestone, medroxy-progesterone, megestrol acetate, norethindrone (also known as norethisterone), norethindrone acetate, norethynodrel, norgesterone, norgestrel, and quingestanol acetate.

Representative androgens include testosterone, testosterone propionate, and methyl testosterone.

Representative drugs useful for the management of diabetes that can be administered by the delivery devices of the invention include insulin, neutral insulin, zinc insulin, dalanated insulin, zinc globin insulin, isophane insulin, protamine insulin, extended zinc insulin, sulfonylurea hypoglycemics, acetohexamide, glypinamide, chlorpropamide, tolazamide, tolbutamide, phenformin, and the like.

Representative of other drug species that can be delivered include penicillin, cephalosporins, erythromycin, lincomycin, tetracycline, streptomycins. bethanidine, clonidine, debrisoquin, amyl nitrite, glyceryl trinitrate, octyl nitrate, isosorbide dinitrate, mannitol hexanitrate, pentaerythritol tetranitrate, theophylline, dopa, methyldopa, norepinephrine, naproxol, metoprolol, aspirin, acid, oxprenolol, and the like. The specific drugs disclosed herein, their dosage unit amount, and their pharmaceutical activity are disclosed in *Cutting's Handbook of Pharmacology*, 6th Ed., 1972, published by Appleton-Century-Crofts, New York, and in *Physicians' Desk Reference*, 34th Ed., 1980, published by Medical Economics Co., Oradell, New Jersey.

The devices of the invention can be manufactured by standard techniques. For example, the bioerodible polymers mixed with release rate modifiers and drugs can be extruded into filaments, spun into fibers, extruded, pressed into shaped articles, solvent film cast, doctor-bladed into thin films, coated by solvent evaporation, coated by using a fluidized bed, compression and transfer molded, and like methods of manufacture. In the process of manufacturing the devices, the materials and ingredients used are predried, and the mixing and fabrication procedures are carried out in an inert, dry atmosphere. The devices made by these processes can be a single matrix, a container with a reservoir, or a number of layers. The devices can be manufactured into various shapes, for example, flat, square, round, tubular, disc, ring, and the like.

The following examples are set forth as representative methods illustrative of the invention. These examples are not to be construed as limiting the scope of the invention, as these and equivalent methods will be apparent to those skilled in the subject art.

Example 1

A bioerodible drug delivery device was prepared as follows: to 90 grams of dry poly(2,2-dioxo-cis/trans-1,4-cyclohexane dimethylene tetrahydrofuran) was added 10 grams of hydrocortisone and the polymer and drug thoroughly blended to form a homogenous composition. The hydrocortisone was previously dried under vacuum at 60 degrees C for at least 8 hours. The hydrocortisone was thoroughly blended with the polymer to yield the composition by heating them to 130 degrees C and mixing them in a blender. The blending was done in a vacuum drybox, and the polymer and drug were blended on a Teflon® sheet heated on a hot plate.

Next, the homogenous composition was pressed into a 10 mil thick sheet using a Carver® press

in a Labconco® drybox having a nitrogen atmosphere. The composition was pressed at 24,000 psi at 130 degrees C between Teflon®-aluminum sheets with the Teflon® facing the polymer-drug composition. After pressing, the sheet was transferred to a vacuum drybox having a helium atmosphere, and a drug delivery device was die-cut with a 5 mm×8 mm oval punch. This device served as a control for determining the amount of drug released over time. The release rate was determined by placing the device in a 30 ml aqueous bath, at 37 degrees C, having a pH of 7.1, obtained by using a 0.1 N phosphate buffer. The release of hydrocortisone was measured by U.V. absorption at 245 m$\mu$.

Example 2

The procedure described in Example 1 was repeated and a drug delivery device was prepared, comprising the polymer, poly(2,2-dioxo-1,6-hexamethylene tetrahydrofuran), 5% by weight hydrocortisone, 12.4% by weight of CaO. A control system was prepared to demonstrate the release pattern of an identical device prepared without the rate controlling agent, CaO. The release of hydrocortisone into a 0.1 molar phosphate buffer having a pH of 7.4 maintained at 37°C is indicated in Figure 6. In Figure 6, the line connecting the circular symbols show a relative release rate of hydrocortisone from the device containing 12.4% CaO which is approximately one-half of the release rate shown by the device made without any CaO (square symbols). The units along the abscissa indicate the absorbance of a specific wave length of ultraviolet light. An additional series of drug delivery devices were prepared containing the same polymer and drug of Example 1, and the erosion rate modifier, calcium oxide in the following weight percents: 0.1%, 0.3%, 1.0%, 3.0%, and 10%. The results for Example 1 and of the additional series of Example 2 are set forth in Figure 7. In Figure 7, A indicates the control of Example 1, B indicates a device containing 0.1% CaO, C indicates a device containing 0.3% CaO, D indicates 1% CaO, E indicates another device containing 1% CaO, F indicates a device containing 3% CaO, and G indicates a device containing 10% CaO.

Example 3

The procedure of Example 1 was followed in this example, with the erodible device made of the same polymer, containing 10% hydrocortisone, and a 10% of an erosion rate modifiers. A series of erosion rate modifiers were used to demonstrate the increased life of the device. The results obtained for the modifiers are set forth in Figure 8, wherein the modifiers are as follows: A indicates a control made without a modifier, H indicates a device containing the modifier calcium dibasic phosphate, I indicates a device containing calcium phosphate, J indicates a device containing calcium oxide, K indicates a device containing magnesium carbonate, L indicates the erosion rate of another device containing 10% magnesium carbonate, and M indicates the erosion rate of a device containing 10% sodium carbonate.

Example 4

The procedure of Example 1 was followed in this example. In this example, an erodible device was prepared comprising 5% hydrocortisone, 10% calcium oxide and 85% poly(2,2-dioxo-cis/trans-1,4-cyclohexane dimethylene tetrahydrofuran), which device exhibits a release rate profile identified as N in Figure 9; and a device comprising 5% hydrocortisone, 5% sodium carbonate, 5% sodium bicarbonate, and 85% poly(2,2-dioxo-cis/trans-1,4-cyclohexane dimethylene tetrahydrofuran), which device exhibits a release rate profile identified as Q in Figure 9.

Example 5

A bioerodible drug delivery device, manufactured as an implant, for releasing a contraceptively effective amount of a steroid over a prolonged period of time was manufactured using the anhydrous drying and process techniques described in Examples 1 through 4. The device of this example was manufactured as follows: first, 70 grams of a bioerodible polymer of the following structure was heated in a commercial blender to 120 degrees C. The polymer had a molecular weight of about 35,000, and the blender had a dry nitrogen atmosphere. Next, 20 grams of dry sodium carbonate was added to the polymer,

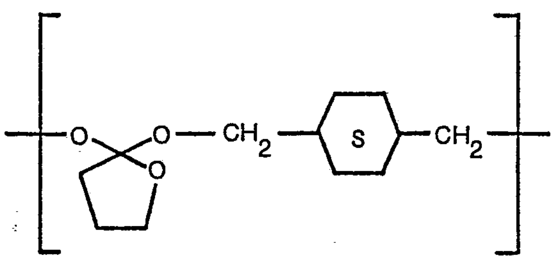

and the two ingredients blended for 5 minutes. The blender mixed the ingredients with an internal stirrer set at 200 resolutions per minute. Then, 10 grams of dry d-norethisterone was added to the blender and the three ingredients blended for 10 minutes to form a homogenous composition.

After blending, the composition was transferred to a mold having a series of cavities shaped as implants. The mold was in a dry box, under a helium atmosphere. The composition was charged into the mold, and pressed into implants under a pressure of 20,000 psi, at 110 degrees C. The implants were shaped as rods 14 mm long by 3 mm in diameter with rounded ends, and they weighed about 125 mg. The implants, when in operation *in vivo*, exhibit a rate of release of about 150 $\mu$g per day of norethisterone over a prolonged period of about 4 months.

Example 6

Three implants, designed for subcutaneous or intramuscular implantation were prepared in a blender, under anhydrous conditions, by separately blending predried ingredients such as, (a) 70 grams of poly(2,2-dioxo-cis/trans-1,4-cyclohexane dimethylene-2-tetrahydrofuran), 10 grams of micronized calcium oxide, and 20 grams of micronized norethindrone; (b) 70 grams of poly(2,2-dioxo-cis/trans-1,4-cyclohexane dimethylene-2-tetrahydrofurna), 5 grams of micronized calcium oxide, and 25 grams of micronized norethindrone; and (c) 75 grams of poly(2,2-dixo-cis/trans-1,4-cyclohexane dimethylene-2-tetrahydrofuran), 5 grams of micronized calcium oxide, and 20 grams of micronized norethindrone. The time of blending was 20 minutes, at 110 degrees C, with a stirrer speed of 25 revolutions per minute, rpm. The three blends were injection molded into implants 14 mm×3 mm, with each weighing about 120 mg. The systems contained 24 to 32 mg of norethindrone, and released about 200 $\mu$g/day of the steroid.

Example 7

The procedures set forth in the above examples were followed for making a series of implants comprising 50 to 80% of poly(2,2-dioxo-cis/trans-1,4-cyclohexane dimethylene-tetrahydrofuran), from 5 to 30% of the erosion rate modifier selected from the group consisting of sodium carbonate and magnesium oxide, and from 5 to 30% of the contraceptive steroid norethindrone. The implant had a presently preferred composition of (a) 70% of poly(2,2-dioxo-cis/trans-1,4-cyclohexane dimethylene-tetrahydrofuran), 10% of sodium carbonate and 20% of norethindrone; (b) 55% of poly(2,2-dioxo-cis/trans-1,4-cyclohexane dimethylene-tetrahydrofuran), 20% of sodium carbonate, and 25% of norethindrone; (c) 50% of poly(2,2-dioxo-cis/trans-1,4-cyclohexane dimethylene-tetrahydrofuran), 20% of sodium carbonate and 30% of norethindrone; (d) 90% of poly(2,2-dioxo-cis/trans-1,4-cyclohexane dimethylene-tetrahydrofuran), 5% sodium carbonate, and 5% norethindrone; (e) 50% of poly(2,2-dioxo-cis/trans-1,4-cyclohexane dimethylene-tetrahydrofuran), 30% of sodium carbonate, and 20% of norethindrone; (f) 70% of poly(2,2-dioxo-cis/trans-1,4-cyclohexane dimethylene-tetrahydrofuran), 20% of magnesium oxide, and 10% of norethindrone; and (g) 60% of poly(2,2-dioxo-cis/trans-1,4-cyclohexane dimethylene-tetrahydrofuran), 10% of magnesium oxide, and 30% of norethindrone.

Example 8

An implant for providing the progestational agent d-norgestrel was prepared using the procedures of the above examples as follows: 20.64 grams of the following polymer:

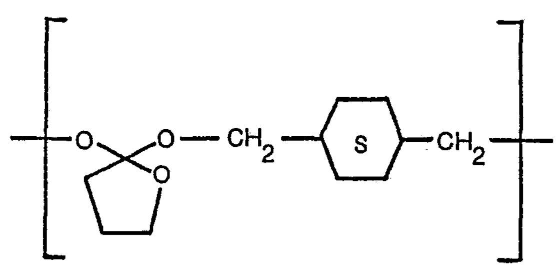

having a molecular weight of 42,473 was heated to 105°C±2°C, on a stainless steel plate in a helium drybox, and then 0.96 grams of d-norgestrel were mixed into the polymer with a spatula. After the polymer and steroid were thoroughly mixed, 2.40 grams of dry sodium carbonate was mixed with the drug polymer mixture. Next, the three component homogenous composition was transferred to a drybox that was previously purged for two hours with dry nitrogen. Then, a mold, having an implant forming cavity, in the drybox was preheated to about 130°C and charged with 8.2 grams of the composition. The mold was changed at 126°C to 130°C, and a pressure of 24,000 psi applied for 15 minutes to form the implant. After the mold cooled, the implants were removed and excess flash was trimmed from the implants. The implants weighed 0.118 grams and they comprised 86% of the polymer, 10% of the modifier, and 4% of the steroid. The implants are useful for maintaining an antifertility state when implanted in the tissue of animals.

Example 9

The procedure of Example 8 was repeated here, with all conditions as set forth, except that implants were made from polymers having a molecular weight of 34,285 and a molecular weight of 33,873.

Example 10

To 26.7 grams of poly(2,2-dioxo-cis/trans-1,4-cyclo-hexane dimethylene tetrahydrofuran), in an Atlantic reactor with 2 mixer blades rotating at 35 rpm, and preheated to 120°C, was added 5.9 grams of d-norgestrel, and the two ingredients mixed for 15 minutes, under a nitrogen atmosphere. Next, 8.8 grams of sodium carbonate was added to the reactor, and the three ingredients blend to produce a composition having a polymer-steroid-modifier composition of 76.5%-16.8%-6.7%. The composition was manufactured in a dry environment and with anhydrous ingredients into implants by following the procedure described above.

Repeating the procedure of the example, the following implants were made: (a) an implant comprising 76.4% poly(2,2-dioxo-cis/trans-1,4-cyclohexane dimethylene tetrahydrofuran), 16.8% levo-norgestrel, and 6.8% sodium bicarbonate; (b) 76.5% poly(2,2-dioxo-cis/trans-1,4-cyclohexane dimethylene tetrahydrofuran), 16.8% levo-norgestrel, and 6.6% of magnesium oxide; (c) an implant comprising 76.6% poly(2,2-dioxo-cis/trans-1,4-cyclohexane dimethylene tetrahydrofuran), 16.8% levo-norgestrel, and 6.7% lithium hydroxide; and (d) 71.0% poly(2,2-dioxo-cis/trans-1,4-cyclohexane dimethylene tetrahydrofuran), 15.6% levo-norgestrel, and 13.4% calcium hydride.

Example 11

The procedure of Example 10 was repeated for making an implant comprising 75% of poly(2,2-dioxo-cis/trans-1,4-cyclohexane dimethylene tetrahydrofuran), 15% d-norgestrel, and 10% magnesium oxide. The implant had a rate of release of 40 μg/day of the d-norgestrel and over a 6 month period. Another implant was prepared comprising 75% of poly(2,2-dioxo-cis/trans-1,4-cyclohexane dimethylene tetrahydrofuran), 15% d-norgestrel, and 10% sodium carbonate. The implant released 40 μg/day of d-norgestrel over a 6 month period.

Example 12

The procedure of Example 10 was followed for producing an implant comprising 80% poly(2,2-dioxo-cis/trans-1,4-cyclohexane dimethylene tetrahydrofuran), 10% insulin and 10% sodium carbonate. The polymer had a molecular weight of 36,400, the implant weighed 20 mg, and the implant released 3.2 units of insulin per day for 14 days.

Example 13

A series of implants were prepared by heating a poly (orthoester) to 90°C±4°C on a Teflon® platen in a nitrogen atmosphere. A modifier was physically mixed into the heated polymer using a stainless steel spatula for 10 minutes, and then, insulin was mixed therein for 10 to 15 minutes. The formulations were transferred to a dry box with a nitrogen atmosphere, and pressed into a 20 mil thick film with a Carvert press at 190°F/20,000 psi for 5 minutes, followed by 5 minutes of cooling. Then, elliptical implants, 5 mm by 8 mm were punched from each film at room temperature in a nitrogen atmosphere. Following this procedure the following implants were prepared: a) 80.2% poly(2,2-dioxo-cis/trans-1,4-cyclohexane dimethylene tetrahydrofuran), 10.2% sodium carbonate, and 9.7% insulin; b) 75.4% poly(2,2-dioxo-cis/trans-1,4-cyclohexane dimethylene tetrahydrofuran), 10.2% sodium acid carbonate, and 14.4% insulin; c) 70% poly(2,2-dioxo-cis/trans-1,4-cyclohexane dimethylene tetrahydrofuran), 10% sodium carbonate, and 20% insulin; and d) 60% poly(2,2-dioxo-cis/trans-1,4-cyclohexane dimethylene tetrahydrofuran), 10% magnesium oxide and 30% insulin. The polymer used for manufacturing the implant had a molecular weight of 41,000 to 43,000, the implants weighed 20 mg, and they released 8 to 20 units of insulin per day and it had a duration of between 7 and 21 days.

Example 14

A number of antifertility implants were made comprising the following ingredients, a polymer of the following structure:

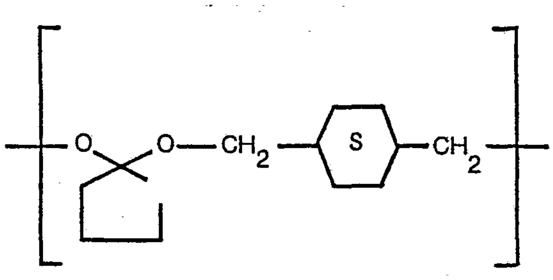

with the implant comprising the following:

| Modifier | d-Norgestrel | Polymer | Mol wt×$10^4$ |
|---|---|---|---|
| 6.7% $Na_2CO_3$ | 16.8% | 76.5% | 4.23 |
| 13.3% $Na_2CO_3$ | 15.6% | 71.1% | 4.25 |
| 6.7% MgO | 16.8% | 76.6% | 4.13 |
| 13.3% MgO | 15.7% | 71.0% | 4.37 |
| 6.7% LiOH | 16.8% | 76.5% | 3.78 |
| 13.3% LiOH | 15.6% | 71.1% | 3.93 |
| 6.7% $CaH_2$ | 16.8% | 76.5% | 3.61 |
| 13.3% $CaH_2$ | 15.6% | 71.0% | 3.92 |
| 6.7% $NaHCO_3$ | 16.8% | 76.5% | 3.62% |
| 13.3% $NaHCO_3$ | 15.6% | 71.1% | 3.52% |

Example 15

The procedure of Example 14 was repeated to provide the implants comprising the following ingredients, expressed as (ratio by weight):

| Modifier | d-Norgestrel | Polymer | Release rate g/day norgestrel |
|---|---|---|---|
| $Na_2CO_3$ (8) | (18) | (82) | 65±4 |
| $Na_2CO_3$ (12) | (18) | (82) | 48±3 |
| $Na_2CO_3$ (6) | (18) | (82) | 62±3 |
| MgO (12) | (18) | (82) | 78±5 |
| MgO (16) | (18) | (82) | 53±2 |

Example 16

Compositions comprising a bioerodible polymer, a modifier, and a drug were prepared under anhydrous conditions and procedures as described in earlier examples as follows: first, a weighed amount of poly(2,2-dioxo-1,6-hexamethylene tetrahydrofuran) was heated to 150°C in a glass beaker, and then a weighed amount of drug was mixed with the polymer. A glass rod was used to stir the polymer and the drug for producing a homogenous mixture. Next, a weighed amount of a polymer erosion rate modifier was added to the beaker, and the three ingredients stirred to produce a homogenous composition. The blending was carried out in a dry box under a nitrogen atmosphere. The following compositions were prepared by this procedure: a) a composition comprising 90% of poly(2,2-dioxo-1,6-hexamethylene tetrahydrofuran), 10% of riboflavin, 5% sodium carbonate and 5% magnesium oxide; b) 45% of poly(2,2-dioxo-1,6-hexamethylene tetrahydrofuran), 45% theophylline and 10% sodium carbonate; c) 87% of poly(2,2-dioxo-1,6-hexamethylene tetrahydrofuran), 8% of hydroxyproline and 5% sodium carbonate d) 50% of poly(2,2-dioxo-1,6-hexa-methylene tetrahydrofuran), 40% of dexamethasone and 10% of sodium carbonate; f) 65% of poly(2,2-dioxo-1,6-hexamethylene tetrahydrofuran), 25% of fluocinolone and 10% of magnesium oxide; g) 70% poly(2,2-dioxo-1,6-hexamethylene tetrahydrofuran), 5% ethinyl estradiol, 15% norethindrone, and 10% sodium carbonate; h) 70% poly(2,2-dioxo-1,6-hexamethylene tetrahydrofuran), 5% mestranol, 20% norethynodrel, and 5% magnesium oxide; and i) 70% poly(2,2-dioxo-1,6-hexamethylene tetrahydrofuran), 4% nitroglycerin, 16% lactose, and 10% sodium carbonate.

Example 17

A drug delivery device for administering a drug at a controlled rate topically to the skin of a human for systemic circulation was made under dry conditions by pressing a composition comprising 50 parts of poly(2,2-dioxo-1,6-hexamethylene tetrahydrofuran), 50 parts of 17-estradiol and 5 parts of sodium bicarbonate was pressed between two aluminum foil sheets into a 20 mil thick film. Then, 2 $cm^2$ topical

delivery devices were punched from the foil sheets, and one sheet was peeled from the device before use. The other sheet served as a backing member for the polymer-estradiol-sodium bicarbonate composition. The composition is tacky, and when placed on the skin of a human, it sticks to the skin. A 2 $cm^2$ device in contact with the skin of a female, produced about a 50 mg per hour increase in urinary excretion of estradiol-conjugates as determined by radio immuno assay after enzymatic hydrolysis, over a normal urinary level of about 50 $\mu$g per hour. An increase of from 2 $cm^2$ to 4 $cm^2$ increased the urine level an additional 25 to 30 $\mu$g per hour. When 5 parts of sodium carbonate were used instead of 5 parts of sodium bicarbonate, the release of estradiol was then halved.

Example 18

A bandage 2 cm by 7 cm for the management of ovulation is prepared under dry and inert conditions by coating one surface of a backing member comprising a polyethylene aluminum foil laminate, which backing member defines the bandage, with a 6 mm thick composition comprising 60 parts of poly(2,2-dioxo-1,6-hexamethylene tetrahydrofuran), 30 parts of norgestrel, 5 parts of Cab-o-sil, and 10 parts of sodium carbonate. The backing member is impermeable to the passage of steroid, and the composition is coated onto the polyethylene surface of the member. The bandage comprising the composition is sticky, and when applied to the skin releases a controlled and continuous amount of steroid over a prolonged period of time.

Example 19

The bandage of Example 18 is prepared with the steroid in the present example comprising a member selected from the group consisting of norethindrone; a mixture of ethinyl estradiol and norethindrone; a mixture of mestranol and norethynodrel; a mixture of ethinyl estradiol and norgestrel; a mixture of mestranol and norethindrone; and a mixture of ethinyl estradiol and dimethisterone.

Example 20

The bandage of 18 is prepared with the backing member additionally comprising a perimeter of a non-irritating pressure sensitive adhesive that surround the steroid, which adhesive is adapted for holding the steroid in intimate contact with the skin, and wherein the adhesive is selected from acrylic adhesives and urethane adhesives, and the steroid is selected from norethindrone; norgestrel; a mixture of ethinyl estradiol and norethindrone; and a mixture of ethinyl estradiol and norgestrel.

Example 21

A series of implants were prepared by heating a poly(orthoester) to 110°C±5°C in a stainless steel vessel, equipped with a stirrer, under a dry nitrogen atmosphere. A modifier and drug were blended into the molten polymer, a process which required less than two minutes. The blends were transferred to a small extruder, and they were transformed into an extrudate shaped as a long rod, 3 mm in diameter. Following this procedure, a number of implants were prepared; all containing 20% progesterone in poly(2,2-dioxo-cis/trans-1,4-cyclohexane dimethylene-tetrahydrofuran). The remaining 80% of the implant were as follows:

| Modifier | Polymer | Mol wt $\times 10^4$ |
|---|---|---|
| 2-phenylcyclopropylamine 5% | 75% | 4.8 |
| Trihydroxytriethylamine 6% | 74% | 3.6 |
| 2,4,6-trimethylpyridine 5% | 75% | 3.8 |
| 2-amino-2-hydroxymethyl-1,3-propanediol 2.5% | 77.5% | 4.0 |
| None | 80% | 3.8 |

The rods were cut into lengths between 1 cm and 2.5 cm and then placed in the subcutaneous tissue of rats. At the end of one month, at least part of all those implants which contained modifiers were found at the site of implantation. The implant which did not contain any erosion rate modifier was not visible on macroscopic examination at the site of implantation. The experiment indicates the amine modifiers retard the rate of erosion and drug delivery from the implants.

Example 22

The procedure of Example 13 was followed for producing an implant comprising 80% poly(2,2-dioxo-cis/trans-1,4-cyclohexane dimethylene-tetrahydrofuran), 16% hydrocortisone, and 4% of a rate controlling modifier. The initial molecular weight of the polymer was 37,000. In vitro release studies were performed in 0.1 molar sodium phosphate buffers. The results for a series of modifiers and the approximate time at which greater than 90% of the drug was released are as follows:

| Modifier | Time |
|---|---|
| $NaH_2PO_4$ | <3 days |
| $Na_2HPO_4$ | 5 days |
| $Na_2PO_4$ | 17 days |
| $MgSO_4$ | 10 days |
| $NaHCO_3$ | 8 days |
| $Na_2CO_3$ | >20 days |
| $Na_2CO_3$+CaO | >20 days |

Example 23

A drug delivery device for administering a drug at a controlled rate into the ano-rectal passageway was made by formulating 50 parts of theophylline, 10- parts of diethylamine and 40 parts of poly(2,2-dioxo-1,6-hexamethylene tetrahydrofuran) in a dry nitrogen atmosphere. A measured sample was placed in the passageway of dogs and plasma samples were taken at 4 hour intervals. The results indicated that 90% of the drug released from the device made with the modifier, was absorbed into the blood system during the first 18 hours after administration. When the rate accelerating modifier was omitted from the device, about 50% of the drug was absorbed during the same time period.

Example 24

The procedure of Example 22 was followed for producing an ocular insert comprising 80% poly(2,2-dioxo-cis/trans-1,4-cyclohexane dimethylene titrahydrofuran), 16% prednisolone acetate and 4% sodium carbonate. The ocular implant was placed in one eye of a test host and it had a prolonged life of 17 days. An ocular implant made without the sodium carbonate modifier was placed in the other eye of the host, and it had a life of about two days duration.

Example 25

A drug delivery device for administering a drug at a controlled rate into a vagina is made by formulating 50 parts of progesterone, 10 parts of diethylamine and 40 parts of poly(2,2-dioxo-1,6-hexamethylene tetrahydrofuran) in a dry nitrogen atmosphere by following the fabrication procedures as described above. The device when inserted in the vagina, administers the steroid over a prolonged period of time.

Example 26

A drug delivery device for administering an anti-ulcer drug to the gastrointestinal tract is made by formulating 50 parts of mepyramine, 10 parts of sodium carbonate and 40 parts of poly(2,2-dioxo-cis/trans-1,4-cyclohexane dimethylene tetrahydrofuran) in a dry inert atmosphere by following the above procedures. The device when administered orally released the drug over time.

Example 27

A series of drug delivery devices are prepared by following the above procedures and using the above erosion rate modifiers, with the polymer in the present example a member selected from the groups as follows: poly(1,1-dioxo-cis/trans-1,4-cyclohexane dimethylene cyclopentane; poly(1,1-dioxo-cis/trans-1,4-cyclohexane dimethylene cyclohexane; poly(1,1-dioxo-1,6-hexamethylene cyclopentane); poly(1,1-dioxo-1,6-hexamethylene cyclopentane; and poly(1,1-dioxo-1,10-decamethylene cyclopentane).

Example 28

Twenty grams of tetracycline, the free base and previously dried at 60°C for 8 hours in vacuum, was thoroughly mixed with 0.1% of the anionic surfactant dioctyl sodium sulfosuccinate, and the antibiotic surfactant mixture blended with 80 grams of poly(2,2-dioxo-cis/trans-1,4-cyclohexane

dimethylene tetrahydrofuran). The polymer was previously heated to 100—120°C, and the mixture mixed therein for 15 to 20 minutes to yield a homogenous composition.

Next, a 20 mil, 3″×3″ film was pressed at 150°F at 1500 psi for 5 minutes, followed by a 5-minute cooling period. Then, ellipsoidal devices 5×8 mm were punched from the cool film. The devices are useful as ocular inserts.

Example 29

To 80 grams of a polymer of the formula, having a molecular weight of 44,000, where n is about 216, and which polymer

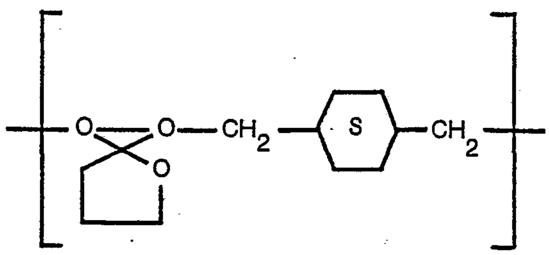

was previously heated to 110—120°C, was added 0.1 g of the neutral surfactant t-octyl-phenoxy-polyoxyethanol, and the polymer surfactant blended to give a homogenous composition. Then, with the composition at the raised temperature, 20 grams of dry tetracycline base was blended into the composition. The blending continued for 15 to 20 minutes.

Next, a 20 mil film was pressed at 150°F/15000 psi for 5 minutes, followed by a 10 minute cooling period. Ocular inserts of elipse shape, 5×8 mm were then punched from the film.

Example 30

A composition, useful as a control for demonstrating the invention, was prepared by blending 20% tetracycline with the polymer illustrated in Example 2. Control inserts, 5×8 mm were prepared as described above.

Examples 31 to 34

Ocular dispensing devices comprising a) 0.006% of benzalkonium chloride, 20% tetracycline, and 80% poly(2,2-dioxo-cis/trans-1,4-cyclohexane dimethylene tetrahydrofuran); b) 0.05% of benzalkonium chloride, 20% of tetracycline, and 80% of poly(2,2-dioxo-cis/trans-1,4-cyclohexane dimethylene tetrahydrofuran; c) 0.1% triethanolamine, 20% tetracycline and 80% poly(2,2-dioxo-cis/trans-1,4-cyclohexande dimethylene tetrahydrofuran; and d) 0.1% benzalkonium chloride, 20% tetracycline, and 20% poly(2,2-dioxo-cis/trans-1,4-cyclohexane dimethylene tetrahydrofuran, were manufactured according to the procedures described in Examples 28 to 30.

The drug release rates were determined for the devices in pH, 0.1 M phosphate buffer, and the drug release profiles presented as weight and percentage of drug released per hour. The release of the drug was at 37°C. The drug release rates were determined by the plot of UV absorbance at 356 nm vs. time in phosphate buffer. The results of the measurements are presented in Table 1. In the table, anionic surfactant indicates dioctyl sodium sulfosuccinate; neutral indicates t-octylphenoxypolyethox-ethanol, BzAC indicates benzalkonium chloride; and TEA indicates triethanolamine.

TABLE 1

| Composition | Rate of drug release (μg/hr) | Lifetime (hrs) |
|---|---|---|
| Control | 120 | 50 |
| 0.1% anionic surfactant | 140 | 50 |
| 0.1% neutral | 94 | 80 |
| 0.006% Cationic (BzAC) | 64 | 100 |
| 0.05% Cationic (BzAC) | 50 | 170 |
| 0.1% Cationic (TEA) | 37 | 115 |
| 0.1% Cationic (BzAC) | 28 | 240 |

The results of the invention are further illustrated in Figures 10 to 16. In Figure 10, the device comprised 20% tetracycline and 80% poly(2,2-dioxo-cis/trans-1,4-cyclohexane dimethylene tetrahydrofuran; in Figure 11 the device comprised 20% tetracycline, 0.1% dioctyl sodium sulfosuccinate and 80% poly(2,2-dioxo-cis/trans-1,4-cyclohexane dimethylene tetrahydrofuran); in Figure 12 the device comprised 20% tetracycline, 0.1% t-octylphenoxy-polyethoxyethanol, and 80% poly(2,2-dioxo-cis/trans-1,4-cyclohexane dimethylene tetrahydrofuran); in Figure 13, the device comprised 20% tetracycline, 0.1% triethanolamine, and 80% poly(2,2-dioxo-cis/trans-1,4-cyclohexane dimethylene tetrahydrofuran); in Figure 14 the device comprised 20% tetracycline, 0.006% benzalkonium chloride, and 80% poly(2,2-dioxo-cis/trans-1,4-cyclohexane dimethylene tetrahydrofuran); in Figure 15 the device comprised 20% tetracycline, 0.05% benzalkonium chloride, and 80% poly(2,2-dioxo-cis/trans-1,4-cyclohexane dimethylene tetrahydrofuran; and in Figure 16, the device comprised 20% tetracycline, 0.1% benzalkonium chloride, and 80% poly(2,2-dioxo-cis/trans-1,4-cyclohexane dimethylene tetrahydrofuran).

## Claims

1. A device for delivering a drug, the device comprising: (A) an erodible polymer of the formula:

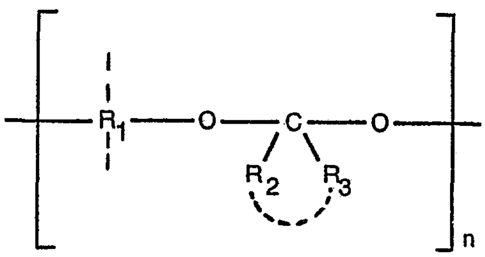

wherein *n* is greater than 10 and wherein (I) $R_1$ is selected from divalent, tribalent and tetravalent radicals consisting of alkylene of 1 to 10 carbons; alkenylene of 2 to 10 carbons; alkyleneoxy alkylene of 2 to 6 carbons; cycloalkylene of 3 to 7 carbons; cycloalkylene of 3 to 7 carbons substituted with an alkyl of 1 to 7 carbons, alkoxy of 1 to 7 carbons, an alkenyl of 2 to 7 carbons, and an alkylene of 1 to 10 carbons; cycloalkenylene of 4 to 7 carbons; optionally substituted with an alkyl of 1 to 7 carbons, an alkoxy of 1 to 7 carbons, alkenyl of 2 to 7 carbons, or an alkylene of 1 to 10 carbons; arylene of 6 to 12 carbons; optionally substituted with an alkyl of 1 to 7 carbons, and alkylene of 1 to 10 carbons; or

wherein (II) $R_2$ and $R_3$ are selected from alkyl of 1 to 7 carbons, alkenyl of 2 to 7 carbons, aryl of 6 to 12 carbons, an oxygen atom covalently bonded to the dioxycarbon atom, and when an oxygen atom $R_2$ and $R_3$ additionally include an alkyloxy of 1 to 7 carbons; alkenyloxy of 2 to 7 carbons; and aryloxy of 6 to 12 carbons; and when only one of $R_2$ and $R_3$ is selected from said member the other $R_2$ and $R_3$ is selected from alkyl of 1 to 7 carbons; alkenyl of 2 to 7 carbons; and aryl of 6 to 12 carbons; or

wherein (III) $R_2$ and $R_3$ are covalently bonded to each other and to the same dioxycarbon atom, with at least one of $R_2$ and $R_3$ being a ring oxygen atom forming a heterocyclic ring of 5 to 8 carbon and oxygen atoms when $R_2$ and $R_3$ are selected from alkylene of 2 to 6 carbons; alkenylene of 2 to 6 carbons; alkylenedioxy of 2 to 5 carbons; alkenylenedieoxy of 2 to 5 carbons; oxa; and a heterocyclic ring of 5 to 8 carbon and oxygen atoms substituted with an alkyl of 1 to 7 carbons, an alkyloxy of 1 to 7 carbons; an alkenyl of 2 to 7 carbons, and an alkenyloxy of 2 to 7 carbons; or

wherein (IV) $R_2$ and $R_3$ are intramolecularly covalently bonded to each other and to the same dioxycarbon atom with at least one of $R_2$ and $R_3$ being a ring oxygen atom forming a fused polycyclic ring of 8 to 12 carbon and oxygen atoms when $R_3$ and $R_3$ are a member selected from aralkylene of 8 to 12 carbons; aralkenylene of 8 to 12 carbons, aryloxy of 8 to 12 carbons; aralkyleneoxy of 8 to 12 carbons; aralkenyleneoxy of 8 to 12 carbons, aralkylenedioxy of 8 to 12 carbons; aralkenylenedioxy of 8 to 12 carbons; oxa; and a fused polycyclic ring of 8 to 12 carbon and oxygen atoms substituted with an alkyl of 1 to 7 carbons, an alkyloxy of 1 to 7 carbons, an alkenyl of 2 to 7 carbons and an alkenyloxy of 2 to 7 carbons; or

wherein (V) $R_2$ and $R_3$ are intramolecularly covalently bonded to each other and to the same dioxycarbon atom to form a 5 to 6 member carbocyclic ring; (B) a beneficial drug in the device; characterized by the inclusion of an erosion rate modifier in the device which acts to either increase or decrease the rate of drug release by controlling the pH at the polymermodifier-fluid-drug interface in use to a desired value different from the pH of the environment, to provide a desired erosion rate for the polymer selected, and correspondingly control the release rate of the drug from the device.

2. The device according to claim 1 wherein the erosion rate modifier is a metal oxide, a salt of electronegative nonmetal oxide, a hydride, a metal, a hydroxide, an amine, an organic acid, an inorganic acid, a Lewis acid, a monobasic acid salt, a polybasic acid salt, a hydroxide of a nonmetal or a surfactant.

3. The device according to Claim 1 wherein the erosion rate modifier is calcium oxide, magnesium

oxide, sodium acid carbonate, sodium carbonate, calcium hydride, lithium hydroxide, sodium phosphate, or sodium hydrogen phosphate.

4. The device according to Claim 1 wherein the erosion rate modifier is trimethylamine, triethylamine, tridecylamine, hydroxylamine, propylamine, or ethylenediamine.

5. The device according to claims 1 to 4 wherein the polymer has the formula

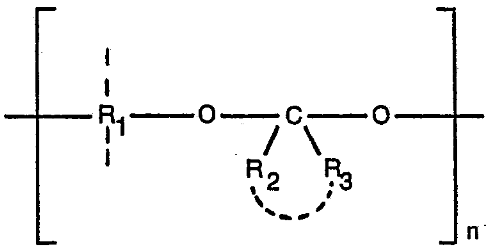

wherein $R_1$ is selected from divalent, trivalent and tetravalent radicals consisting of alkylene of 1 to 10 carbons; alkenylene of 2 to 10 carbons; alkyleneoxy of 2 to 6 carbons; cycloalkylene of 3 to 7 carbons; cycloalkylene of 3 to 7 carbons substituted with an alkyl of 1 to 7 carbons, alkoxy of 1 to 7 carbons, an alkenyl of 2 to 7 carbons, and an alkylene of 1 to 10 carbons; cycloalkenylene of 4 to 7 carbons; cycloalkenylene of 4 to 7 carbons substituted with an alkyl of 1 to 7 carbons, an alkoxy of 1 to 7 carbons, alkenyl of 2 to 7 carbons, and an alkylene of 1 to 10 carbons; arylene of 6 to 12 carbons; arylene of 6 to 12 carbons substituted with an alkyl of 1 to 7 carbons, alkyloxy of 1 to 7 carbons, an alkenyl of 2 to 7 carbons and an alkylene of 1 to 10 carbons; wherein $R_2$ and $R_3$ when taken together form a 5 to 8 member heterocycyclic ring selected from dioxolanyl; dioxanyl; dioxepanyl; dioxocanyl; dioxonanyl; tetrahydrofuranyl; dihydrofuranyl; furyl; pyranyl; ocanyl; and oxepanyl.

6. The device for delivering the beneficial drug over a period of time according to claim 5 wherein the polymer is poly(2,2-dioxo-cis/trans-1,4-cyclohexane dimethylene tetrahydrofuran).

7. The device for delivering the beneficial drug over a period of time according to claim 5 wherein the drug is poly(2,2-dioxo-1,6-hexamethylene tetrahydrofuran).

8. The device according to claim 1 wherein the polymer is defined by the following structural formula:

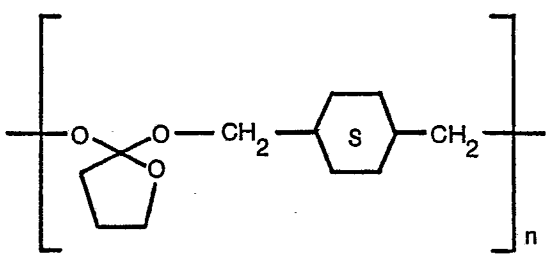

wherein S means saturated and the drug is a hormone selected from cortical, androgenic, estrogenic and progestational hormones; and wherein the erosion rate modifier is selected from metal oxides, salts of nonmetal oxides, hydrides, hydroxides, acids, monobasic, and polybasic salts, acidic salts, amines and mixtures thereof.

9. The device according to claim 1, wherein the surfactant is an anionic or cationic surfactant.

10. A composition of matter comprising a bioerodible polymer of the general structure:

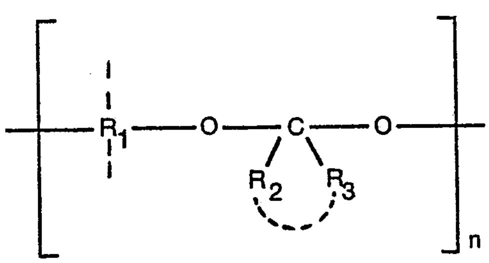

wherein $R_1$ is selected from divalent, trivalent and tetravalent radicals consisting of alkylene of 1 to 10 carbons; alkenylene of 2 to 10 carbons; alkyleneoxy of 2 to 6 carbons; cycloalkylene of 3 to 7 carbons; cycloalkylene of 3 to 7 carbons substituted with an alkyl of 1 to 7 carbons, alkoxy of 1 to 7 carbons, an alkenyl of 2 to 7 carbons, and an alkylene of 1 to 10 carbons; cycloalkenylene of 4 to 7 carbons; cycloalkylene of 4 to 7 carbons substituted with an alkyl of 1 to 7 carbons, an alkoxy of 1 to 7 carbons, an alkenyl of 2 to 7 carbons, and an alkylene of 1 to 10 carbons; arylene of 6 to 12 carbons; arylene of

6 to 12 carbons substituted with an alkyl of 1 to 7 carbons, alkoxy of 1 to 7 carbons, an alkenyl of 2 to 7 carbons, and an alkylene of 1 to 10 carbons; wherein $R_2$ and $R_3$ are taken together to form a 5 to 8 member heterocyclic ring selected from dioxolanyl; dioxanyl; dioxepanyl; dioxonanyl; tetrahydrofuranyl; dihydrofuranyl; furyl; pyranyl; ocanyl; and oxepanyl; characterized by the inclusion of an erosion rate modifier which acts to either increase or decrease the rate or drug release by controlling the pH at the polymer-modifier-fluid-drug interface in use to a desired value different from the pH of the environment, to provide a desired erosion rate for the polymer selected.

**Revendications**

1. Dispositif pour l'apport d'un médicament, comprenant:

(A) une matière polymère érodable de formule:

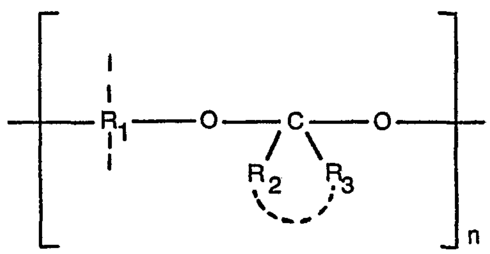

dans laquelle n est un nombre supérieur à 10, (I) $R_1$ est choisi parmi des radicaux divalents, trivalents et tétravalents comprenant des alkylènes en $C_1$ à $C_{10}$; des alcénylènes en $C_2$ à $C_{10}$; des alkylène-oxy-alkylènes en $C_2$ à $C_6$; des cycloalkylènes en $C_3$ à $C_7$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; des cycloalcénylènes en $C_4$ à $C_7$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; et des arylènes en $C_6$ à $C_{12}$ éventuellement substitués par un alkyle en $C_1$ à $C_7$ ou un alkylène en $C_1$ à $C_{10}$; et

(II) $R_2$ et $R_3$ sont choisis chacun parmi des alkyles en $C_1$ à $C_7$, des alcényles en $C_2$ à $C_7$, des aryles en $C_6$ à $C_{12}$, un atome d'oxygène en liaison covalente avec l'atome de carbone lié aux deux oxygènes, avec un atome d'oxygène $R_2$ et $R_3$ pouvant être aussi chacun un alkyloxy en $C_1$ à $C_7$, un alcényloxy en $C_2$ à $C_7$ ou un aryloxy en $C_6$ à $C_{12}$; et si parmi $R_2$ et $R_3$ l'un seulement répond à cette définition, l'autre est choisi parmi des alkyles en $C_1$ à $C_7$, des alcényles en $C_2$ à $C_7$ et des aryles en $C_6$ à $C_{12}$; ou bien

(III) $R_2$ et $R_3$ sont en liaison covalente mutuelle et avec l'atome de carbone lié aux deux oxygènes, l'un d'eux au moins étant un atome d'oxygène formant un hétérocycle avec de 5 à atomes de carbone et d'oxygène si $R_2$ et $R_3$ sont des alkylènes en $C_2$ à $C_6$; des alcénylènes en $C_2$ à $C_6$; des alkylène-dioxy en $C_2$ à $C_5$; des alcénylène-dioxy en $C_2$ à $C_5$ ou des radicaux oxa; ou un hétérocycle ayant de 5 à 8 atomes de carbone et d'oxygène, substitué par un alkyle en $C_1$ à $C_7$, un alkyloxy en $C_1$ à $C_7$, un alcényle en $C_2$ à $C_7$ ou un alcényloxy en $C_2$ à $C_7$; ou bien

(IV) $R_2$ et $R_3$ sont en liaison covalente intramoléculaire mutuelle et avec l'atome de carbone lié aux deux oxygênes, l'un d'eux au moins étant un atome d'oxygène formant un système polycyclique condensé avec de 8 à 12 atomes de carbone et d'oxygène si $R_2$ et $R_3$ sont des aralkylènes en $C_8$ à $C_{12}$; des aralcénylènes en $C_8$ à $C_{12}$; des aryloxy en $C_8$ à $C_{12}$; des aralkylène-oxy en $C_8$ à $C_{12}$; des aralcénylèneoxy en $C_8$ à $C_{12}$; des aralkylène-dioxy en $C_8$ à $C_{12}$; des aralcénylène-dioxy en $C_8$ à $C_{12}$ ou des radicaux oxa; ou un système polycyclique condensé ayant de 8 à 12 atomes de carbone et d'oxygène, substitué par un alkyle en $C_1$ à $C_7$, un alkyloxy en $C_1$ à $C_7$, un alcényle en $C_2$ à $C_7$ ou un alcényloxy en $C_2$ à $C_7$; ou encore

(V) $R_2$ et $R_3$ spnt en liaison covalente intramoléculaire mutuelle et avec l'atome de carbone lié au deux oxygènes en formant un système carbocyclique pentagonal ou hexagonal; avec

(B) un médicament incorporé dans ce dispositif; dispositif caractérisé en ce qu'il comprend aussi un agent modifiant la vitesse d'érosion de la matière polymère, qui agit pour élever ou abaisser la vitesse de libération du médicament en réglant le pH, à l'interface polymèreagent modifiant-liquide-médicament, à une valeur voulue différente du pH du milieu environnant, de manière à donner une vitesse d'érosion voulue pour le polymère choisi et permettre ainsi de régler la vitesse de sortie du médicament du dispositif.

2. Dispositif selon la revendication 1 dans lequel l'agent modifiant la vitesse d'érosion est un oxyde de métal, un sel d'un oxyde de métalloïde électronégatif, un hydrure, un métal, un hydroxyde, une amine, un acide organique ou minéral, un acide de Lewis, un sel d'un monoacide ou d'un polyacide, un hydroxyde de métalloïde ou un agent surfactif.

3. Dispositif selon la revendication 1 dans lequel l'agent modifiant la vitesse d'érosion est l'oxyde de calcium ou de magnésium, l'hydrogéno-carbonate de sodium ou le carbonate de sodium, l'hydrure de calcium, l'hydroxyde de lithium, le phosphate de sodium ou l'hydrogéno-phosphate de sodium.

4. Dispositif selon la revendication 1 dans lequel l'agent modifiant la vitesse d'érosion est la

triméthylamine, la triéthylamine, la tridécylamine, l'hydroxylamine, la propylamine ou l'éthylène-diamine.

5. Dispositif selon la revendication 1 dans lequel la matière polymère est représentée par la formule:

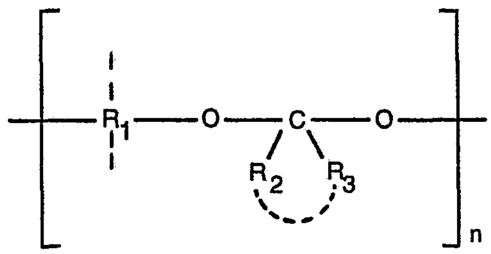

dans laquelle $R_1$ est choisi parmi des radicaux divalents, trivalents et tétravalents comprenant des alkylènes en $C_1$ à $C_{10}$; des alcénylènes en $C_2$ à $C_{10}$; des alkylène-oxy en $C_2$ à $C_6$; des cycloalkylènes en $C_3$ à $C_7$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; des cyclo-alcénylènes en $C_4$ à $C_7$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; et des arylènes en $C_6$ à $C_{12}$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; et $R_2$ et $R_3$ forment ensemble un hétérocycle pentagonal à octogonal choisi parmi les suivants: dioxolanyle, dioxanyle, dioxépanyle, dioxocanyle, dioxonanyle, tétrahydrofurannyle, dihydrofurannyle, furyle, pyrannyle, oxocanyle et oxépanyle.

6. Dispositif selon la revendication 5 dans lequel la matière polymère est le poly(2,2-dioxo-cis/trans-1,4-cyclohexane-diméthylène-tétrahydrofuranne).

7. Dispositif selon la revendication 5 dans lequel la matère polymère est le poly(2,2-dioxo-1,6-hexaméthylène-tétrahydrofuranne).

8. Dispositif selon la revendication 1 dans lequel la matière polymère a la formule suivante:

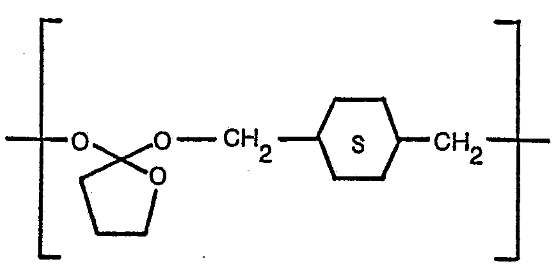

la lettre S signifiant "saturé", le médicament est une hormone choisie parmi des hormones corticoïdes, androgènes, oestrogènes et progestatives, et l'agent modifiant la vitesse d'érosion est choisi parmi des oxydes de métaux, des sels d'oxydes de métalloïdes, des hydrures, des hydroxydes, des acides, des sels de mono-acides et de polyacides, des sels acides, des amines et des mélanges de ces agents.

9. Dispositif selon la revendication 1 dans lequel l'agent surfactif est un surfactif anionique ou cationique.

10. Composition comprenant un polymère bioérodable de formule générale:

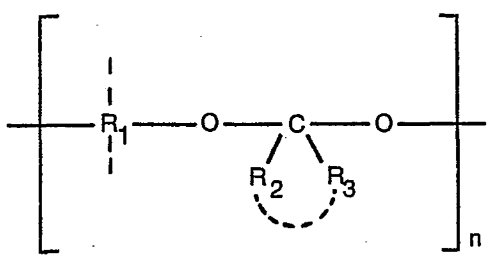

dans laquelle $R_1$ est choisi parmi des radicaux divalents, trivalents et tétravalents comprenant des alkylènes en $C_1$ à $C_{10}$; des alcénylènes en $C_2$ à $C_{10}$; des alkylèneoxy en $C_2$ à $C_6$; des cycloalkylènes en $C_3$ à $C_7$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; des cycloalcénylènes en $C_4$ à $C_7$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; et des arylènes en $C_6$ à $C_{12}$ éventuellement substitués par un alkyle en $C_1$ à $C_7$, un alcoxy en $C_1$ à $C_7$, un alcényle en $C_2$ à $C_7$ ou un alkylène en $C_1$ à $C_{10}$; et $R_2$ et $R_4$ forment ensemble un hétérocycle pentagonal à octogonal choisi parmi

les suivants: dioxolanyle, dioxanyle, dioxépanyle, dioxonanyle, tétrahydrofurannyle, dihydrofurannyle, furyle, pyrannyle, oxocanyle et oxépanyle, composition caractérisée en ce qu'elle comprend également un agent modifiant la vitesse d'érosion du polymère, qui agit pour élever ou abaisser la vitesse de libération d'un médicament contenu dans la composition en réglant le pH, à l'interface polymère-agent modifiant-liquide-médicament, à une valeur voulue différente du pH du milieu environnant, ce qui donne la vitesse d'érosion voulue pour le polymère choisi.

**Patentansprüche**

1. Vorrichtung zur Abgabe eines Wirkstoffs, wobei die Vorrichtung umfaßt:

A) ein abbaubares Polymer der Formel

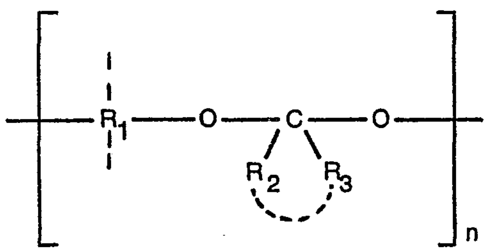

in der n größer ist als 10 und in der (I) $R_1$ ausgewählt ist aus zweiwertigen, dreiwertigen und vierwertigen Resten, bestehend aus Alkylen mit 1 bis 10 Kohlenstoffatomen, Alkenylen mit 2 bis 10 Kohlenstoffatomen; Alkylenoxy alkylen mit 2 bis 6 Kohlenstoffatomen, Cycloalkylen mit 3 bis 7 Kohlenstoffatomen; Cycloalkylen mit 3 bis 7 Kohlenstoffatomen, substituiert mit einem Alkyl mit 1 bis 7 Kohlenstoffatomen, Alkoxy mit 1 bis 7 Kohlenstoffatomen, einem Alkenyl mit 2 bis 7 Kohlenstoffatomen und (oder) einem Alkylen mit 1 bis 10 Kohlenstoffatomen; Cycloalkenylen mit 4 bis 7 Kohlenstoffatomen, gegebenenfalls substituiert mit einem Alkyl mit 1 bis 7 Kohlenstoffatomen, einem Alkoxy mit 1 bis 7 Kohlenstoffatomen, einem Alkenyl mit 2 bis 7 Kohlenstoffatomen oder einem Alkylen mit 1 bis 10 Kohlenstoffatomen; Arylen mit 6 bis 12 Kohlenstoffatomen, gegebenenfalls substituiert mit einem Alkyl mit 1 bis 7 Kohlenstoffatomen und (oder) einem Alkylen mit 1 bis 10 Kohlenstoffatomen oder

wobei (II) $R_2$ und $R_3$ ausgewählt sind aus Alkyl mit 1 bis 7 Kohlenstoffatomen; Alkenyl mit 2 bis 7 Kohlenstoffatomen; Aryl mit 6 bis 12 Kohlenstoffatomen; einem kovalent an das Dioxy-Kohlenstoffatom gebundenen Sauerstoffatom und wenn ein Sauerstoffatom (vorhanden ist) enthalten $R_2$ und $R_3$ zusätzlich ein Alkyloxy mit 1 bis 7 Kohlenstoffatomen; Alkenyloxy mit 2 bis 7 Kohlenstoffatomen und (oder) Aryloxy mit 6 bis 12 Kohlenstoffatomen und wenn nur einer der Reste $R_2$ und $R_3$ aus dieser Gruppe ausgewählt ist, ist der andere Rest $R_2$ oder $R_3$ ausgewählt aus Alkyl mit 1 bis 7 Kohlenstoffatomen, Alkenyl mit 2 bis 7 Kohlenstoffatomen und Aryl mit 6 bis 12 Kohlenstoffatomen oder

wobei (III) $R_2$ und $R_3$ kovalent miteinander und mit dem gleichen Dioxy-Kohlenstoffatom gebunden sind, wobei zumindest einer der Reste $R_2$ und $R_3$ ein Ring-Sauerstoffatom darstellt, das einen heterocyclischen Ring mit 5 bis 8 Kohlenstoff- und Sauerstoffatomen bildet, wenn $R_2$ und $R_3$ ausgewählt sind aus Alkylen mit 2 bis 6 Kohlenstoffatomen; Alkenylen mit 2 bis 6 Kohlenstoffatomen; Alkylendioxy mit 2 bis 5 Kohlenstoffatomen; Alkenylendioxy mit 2 bis 5 Kohlenstoffatomen; Oxa und einem heterocyclischen Ring mit 5 bis 8 Kohlenstoff- und Sauerstoffatomen, substituiert durch ein Alkyl mit 1 bis 7 Kohlenstoffatomen, ein Alkoxy mit 1 bis 7 Kohlenstoffatomen, ein Alkenyl mit 2 bis 7 Kohlenstoffatomen und (oder) ein Alkenyloxy mit 2 bis 7 Kohlenstoffatomen oder

wobei (IV) $R_2$ und $R_3$ intramolekular, kovalent miteinander und mit dem gleichen Dioxy-Kohlenstoffatom gebunden sind, wobei zumindest einer der Reste $R_2$ und $R_3$ ein Ring-Sauerstoffatom bedeutet, das ein kondensiertes polycyclisches Ringsystem mit 8 bis 12 Kohlenstoff- und Sauerstoffatomen bildet, wenn $R_2$ und $R_3$ ausgewählt sind aus Aralkylen mit 8 bis 12 Kohlenstoffatomen, Aralkenylen mit 8 bis 12 Kohlenstoffatomen, Aryloxy mit 8 bis 12 Kohlenstoffatomen, Aralkylenoxy mit 8 bis 12 Kohlenstoffatomen, Aralkenylenoxy mit 2 bis 8 Kohlenstoffatomen, Aralkylendioxy mit 8 bis 12 Kohlenstoffatomen, Aralkenylendioxy mit 8 bis 12 Kohlenstoffatomen, Oxa und einem kondensierten polycyclischen Ring mit 8 bis 12 Kohlenstoff- und Sauerstoffatomen, substituiert mit einem Alkyl mit 1 bis 7 Kohlenstoffatomen, einem Alkoxy mit 1 bis 7 Kohlenstoffatomen, einem Alkenyl mit 2 bis 7 Kohlenstoffatomen und (oder) einem Alkenyloxy mit 2 bis 7 Kohlenstoffatomen oder

wobei (V) $R_2$ und $R_3$ intramolekular kovalent miteinander und mit dem gleichen Dioxy-Kohlenstoffatom gebunden sind unter Bildung eines 5- bis 6-gliedrigen carbocyclischen Ringes

B) einen Wirkstoff in der Vorrichtung gekennzeichnet durch den Einschluß eines die Abbaugeschwindigkeit modifizierenden Mittels in der Vorrichtung, das dazu dient, die Abgabegeschwindigkeit des Wirkstoffs zu erhöhen oder zu verringern durch Einstellung des pH-Wertes an der Grenzfläche Polymer-Modifiziermittel-Flüssigkeit-Wirkstoff bei der Anwendung auf einen gewünschten Wert, der sich von dem pH-Wert der Umgebung unterscheidet, um eine gewünschte Abbaugeschwindigkeit für das

gewählte Polymer zu erzielen und entsprechend die Abgabegeschwindigkeit des Wirkstoffs aus der Vorrichtung zu steuern.

2. Vorrichtung nach Anspruch 1, wobei das die Abbaugeschwindigkeit modifizierende Mittel ein Metalloxid, ein Salz eines elektronegativen Nichtmetalloxids, ein Hydrid, ein Metall, ein Hydroxid, ein Amin, eine organische Säure, eine anorganische Säure, eine Lewissäure, ein einbasisches saures Salz, ein mehrbasisches saures Salz, ein Hydroxid eines Nichtmetalls oder ein grenzflächenaktives Mittel ist.

3. Vorrichtung nach Anspruch 1, wobei das Mittel zur Steuerung der Abbaugeschwindigkeit Calciumoxid, Magnesiumoxid, Natriumbicarbonat, Natriumcarbonat, Calciumhydrid, Lithiumhydroxid, Natriumphosphat oder Natriumhydrogenphosphat ist.

4. Vorrichtung nach Anspruch 1, wobei das die Abbaugeschwindigkeit modifizierende Mittel Trimethylamin, Triethylamin, Tridecylamin, Hydroxylamin, Propylamin oder Ethylendiamin ist.

5. Vorrichtung nach Anspruch 1 bis 4, wobei das Polymer die Formel besitzt

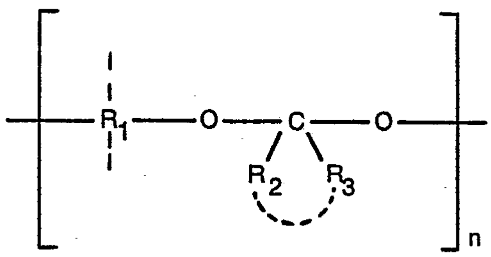

wobei $R_1$ ausgewählt ist aus zweiwertigen, dreiwertigen und vierwertigen Resten, bestehend aus Alkylen mit 1 bis 10 Kohlenstoffatomen; Alkenylen mit 2 bis 10 Kohlenstoffatomen; Alkylenoxy mit 2 bis 6 Kohlenstoffatomen; Cycloalkylen mit 3 bis 7 Kohlenstoffatomen; Cycloalkylen mit 3 bis 7 Kohlenstoffatomen und substituiert mit einem Alkyl mit 1 bis 7 Kohlenstoffatomen, Alkoxy mit 1 bis 7 Kohlenstoffatomen, einem Alkenyl mit 2 bis 7 Kohlenstoffatomen und (oder) einem Alkylen mit 1 bis 10 Kohlenstoffatomen; Cycloalkenylen mit 4 bis 7 Kohlenstoffatomen; Cycloalkenylen mit 4 bis 7 Kohlenstoffatomen und substituiert mit einem Alkyl mit 1 bis 7 Kohlenstoffatomen, einem Alkoxy mit 1 bis 7 Kohlenstoffatomen, Alkenyl mit 2 bis 7 Kohlenstoffatomen und (oder) einem Alkylen mit 1 bis 10 Kohlenstoffatomen; Arylen mit 6 bis 12 Kohlenstoffatomen; Arylen mit 6 bis 12 Kohlenstoffatomen und substituiert mit einem Alkyl mit 1 bis 7 Kohlenstoffatomen, Alkoxy mit 1 bis 7 Kohlenstoffatomen, einem Alkenyl mit 2 bis 7 Kohlenstoffatomen und (oder) einem Alkylen mit 1 bis 10 Kohlenstoffatomen, wobei $R_2$ und $R_3$, wenn sie zusammengenommen werden, einen 5- bis 8-gliedrigen heterocyclischen Ring bilden ausgewählt aus Dioxolanyl, Dioxanyl, Dioxepanyl, Dioxocanyl, Dioxonanyl, Tetrahydrofuranyl, Dihydrofuranyl, Furyl, Pyranyl, Ocanyl und Oxepanyl.

6. Vorrichtung zur Abgabe des Wirkstoffs über einen Zeitraum nach Anspruch 5, wobei das Polymer Poly(2,2-dioxo-cis/trans-1,4-cyclohexan-dimethylen-tetrahydrofuran) ist.

7. Vorrichtung zur Abgabe des Wirkstoffs über einen Zeitraum nach Anspruch 5, wobei der Wirkstoff Poly(2,2-dioxo-1,6-hexamethylen-tetrahydrofuran) ist.

8. Vorrichtung nach Anspruch 1, wobei das Polymer definiert ist durch die folgende Strukturformel

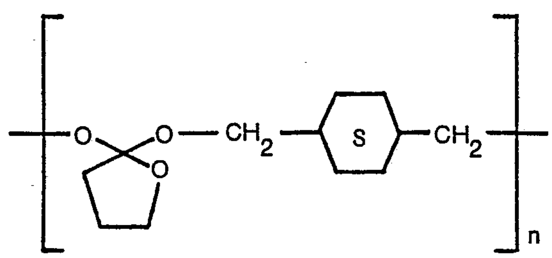

wobei S die Sättigung des Rings angibt und der Wirkstoff ein Hormon ist, ausgewählt aus cortikoiden, antrogenen, östrogenen und progestativen Hormonen, und wobei das Mittel zur Modifizierung der Abbaugeschwindigkeit ausgewählt ist aus Metalloxiden, Salzen von Nichtmetalloxiden, Hydriden, Hydroxiden, Säuren, einbasischen und mehrbasischen Salzen, sauren Salzen, Aminen und deren Gemischen.

9. Vorrichtung nach Anspruch 1, wobei das grenzflächenaktive Mittel ein anionisches oder kationisches grenzflächenaktives Mittel ist.

10. Mittel, umfassend ein biologisch abbaubares Polymer der allgemeinen Formel

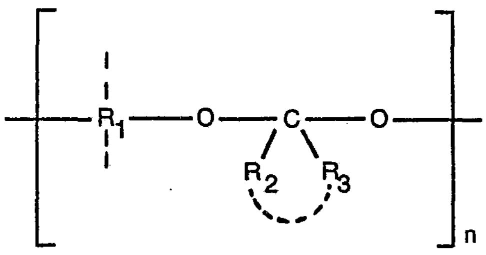

wobei $R_1$ ausgewählt ist aus zweiwertigen, dreiwertigen und vierwertigen Resten, bestehend aus Alkylen mit 1 bis 10 Kohlenstofatomen; Alkenylen mit 2 bis 10 Kohlenstoffatomen; Alkylenoxy mit 2 bis 6 Kohlenstoffatomen; Cycloalkylen mit 3 bis 7 Kohlenstoffatomen; Cycloalkylen mit 3 bis 7 Kohlenstoffatomen und substituiert mit einem Alkyl mit 1 bis 7 Kohlenstoffatomen, Alkoxy mit 1 bis 7 Kohlenstoffatomen, einem Alkenyl mit 2 bis 7 Kohlenstoffatomen und (oder) einem Alkylen mit 1 bis 10 Kohlenstoffatomen; Cycloalkenylen mit 4 bis 7 Kohlenstoffatomen; Cycloalkenylen mit 4 bis 7 Kohlenstoffatomen und substituiert mit einem Alkyl mit 1 bis 7 Kohlenstoffatomen, einem Alkoxy mit 1 bis 7 Kohlenstoffatomen, Alkenyl mit 2 bis 7 Kohlenstoffatomen und (oder) einem Alkylen mit 1 bis 10 Kohlenstoffatomen; Arylen mit 6 bis 12 Kohlenstoffatomen, Arylen mit 6 bis 12 Kohlenstoffatomen und substituiert mit einem Alkyl mit 1 bis 7 Kohlenstoffatomen, Alkoxy mit 1 bis 7 Kohlenstoffatomen, einem Alkenyl mit 2 bis 7 Kohlenstoffatomen und (oder) einem Alkylen mit 1 bis 10 Kohlenstoffatomen, wobei $R_2$ und $R_3$, zusammengenommen einen 5- bis 8-gliedrigen heterocyclischen Ring bilden, ausgewählt aus Dioxolanyl, Dioxanyl, Dioxepanyl, Dioxoanyl, Tetrahydrofuranyl, Dihydrofuranyl, Furyl, Pyranyl, Ocanyl und Oxepanyl gekennzeichnet durch den Einschluß eines die Abbaugeschwindigkeit modifizierenden Mittels das dazu dient, die Abgabegeschwindigkeit des Wirkstoffs zu erhöhen oder zu verringern durch Einstellung des pH-Wertes an der Grenzfläche Polymer-Modifiziermittel-Flüssigkeit-Wirkstoff bei der Anwendung auf einen gewünschten Wert, der sich von dem pH-Wert der Umgebung unterscheidet, um eine gewünschte Abbaugeschwindigkeit für das gewählte Polymer zu erzielen.

FIG.1

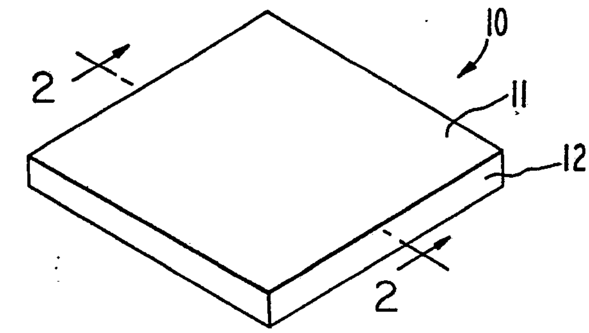


FIG.2

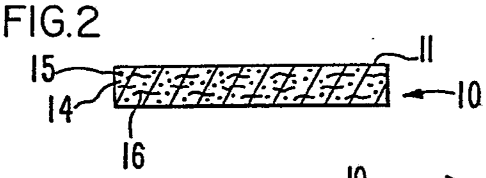


FIG.3

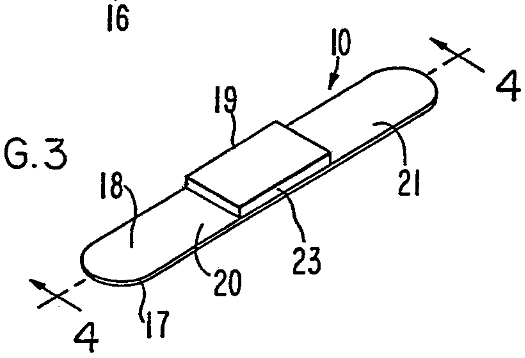


FIG.4

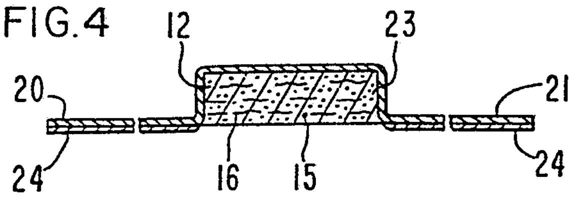


FIG.5

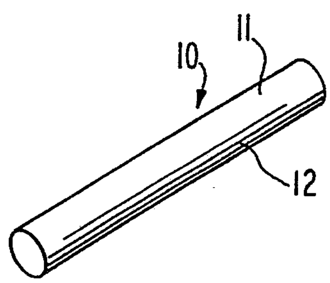

FIG.6

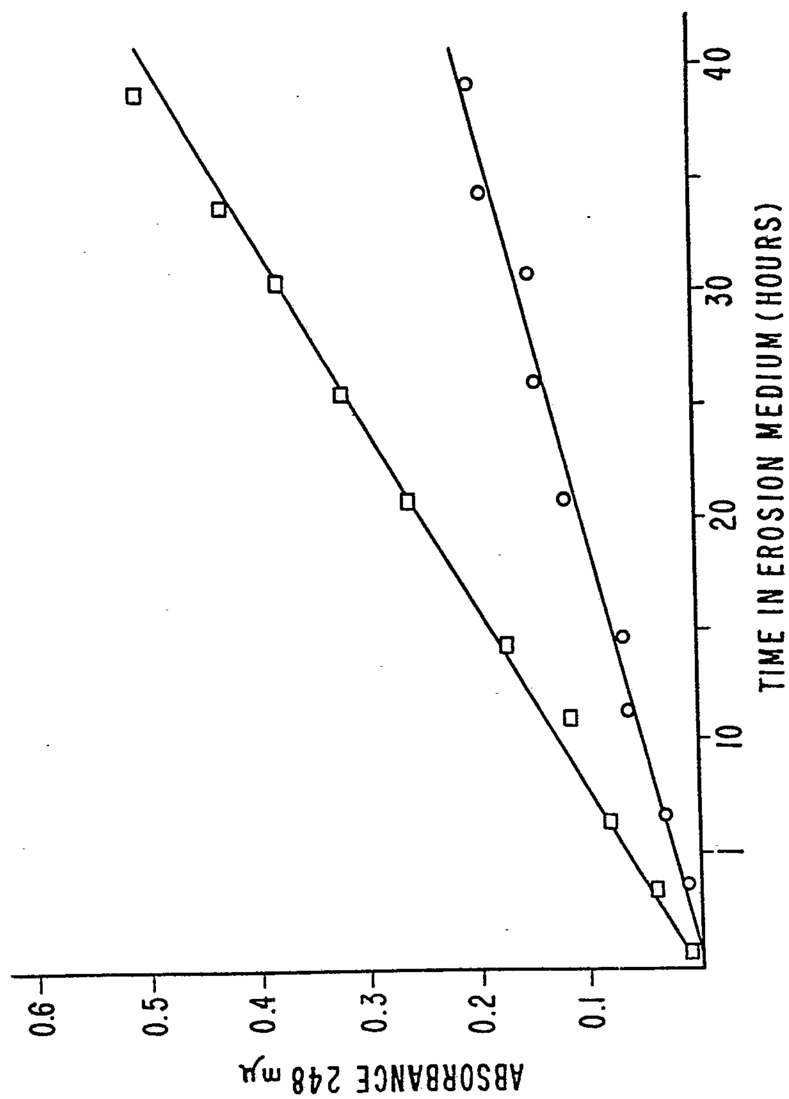
ABSORBANCE 248 mμ
0.1
0.2
0.3
0.4
0.5
0.6
TIME IN EROSION MEDIUM (HOURS)
10
20
30
40

FIG. 7

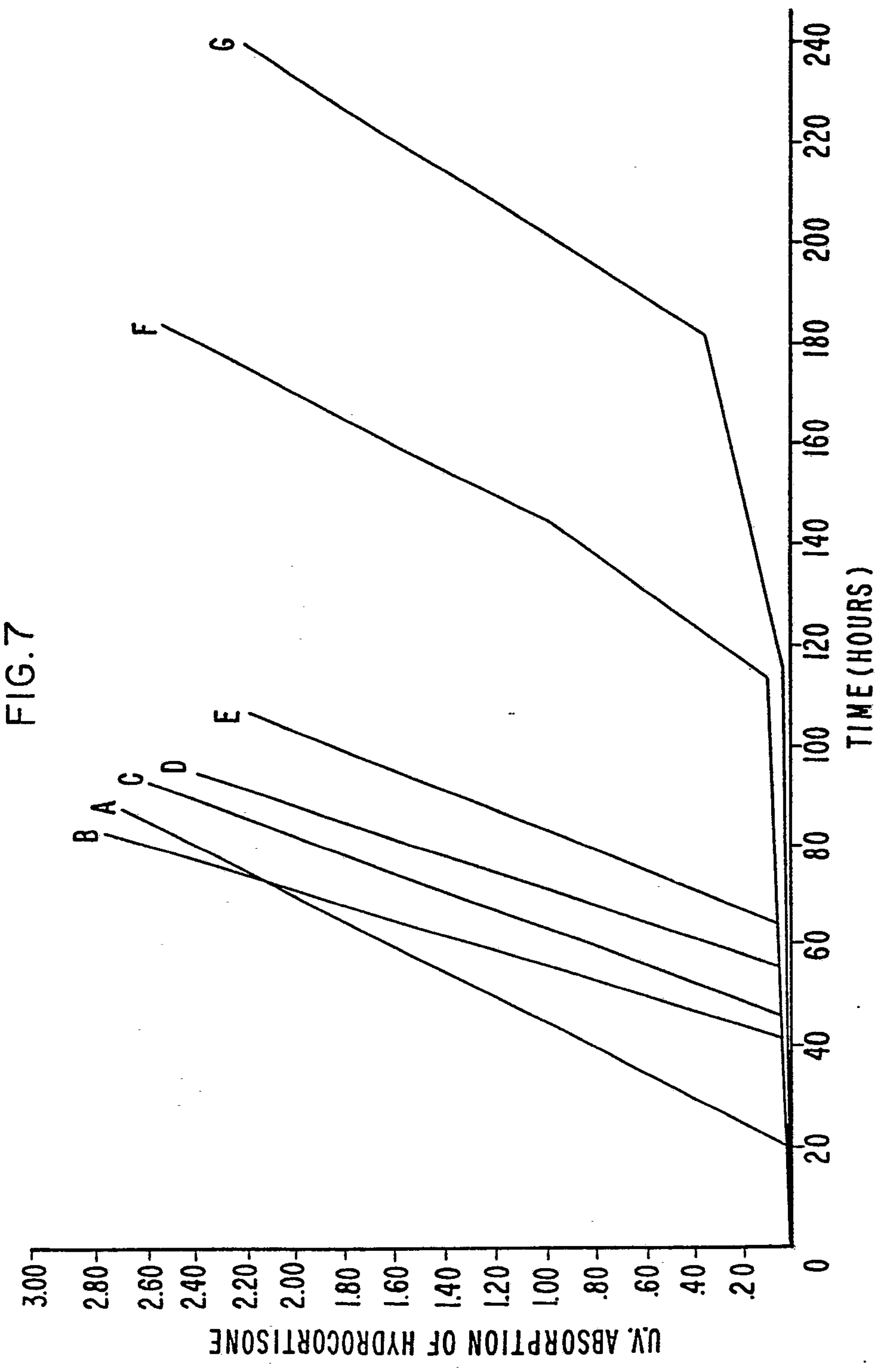
U.V. ABSORPTION OF HYDROCORTISONE
3.00
2.80
2.60
2.40
2.20
2.00
1.80
1.60
1.40
1.20
1.00
.80
.60
.40
2.0
0
20
40
60
80
100
120
140
160
180
200
220
240
TIME (HOURS)
A
B
C
D
E
F
G

FIG.8

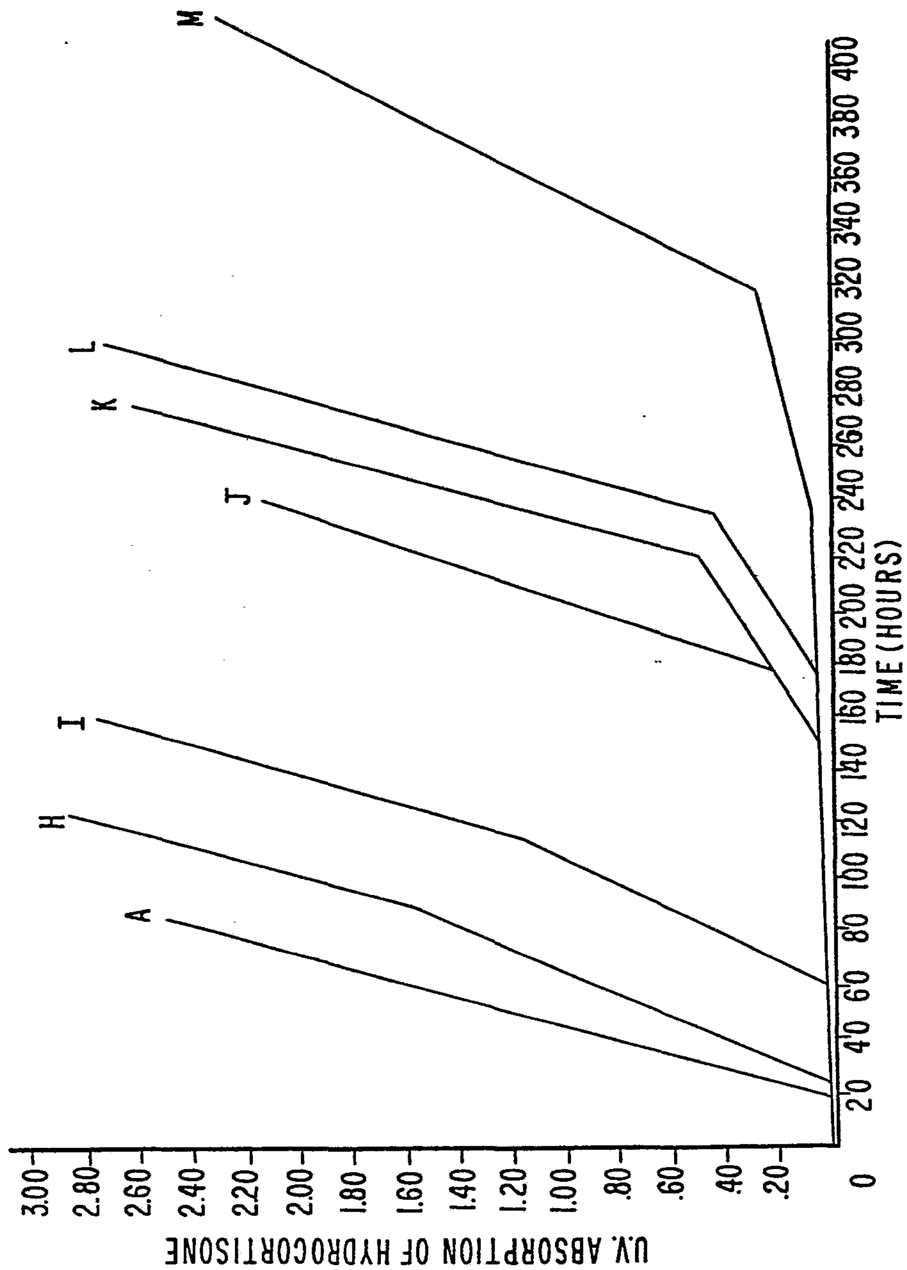
A
H
I
J
K
L
M
U.V. ABSORPTION OF HYDROCORTISONE
3.00
2.80
2.60
2.40
2.20
2.00
1.80
1.60
1.40
1.20
1.00
.80
.60
.40
.20
0
20 40 60 80 100 120 140 160 180 200 220 240 260 280 300 320 340 360 380 400
TIME (HOURS)

FIG.9

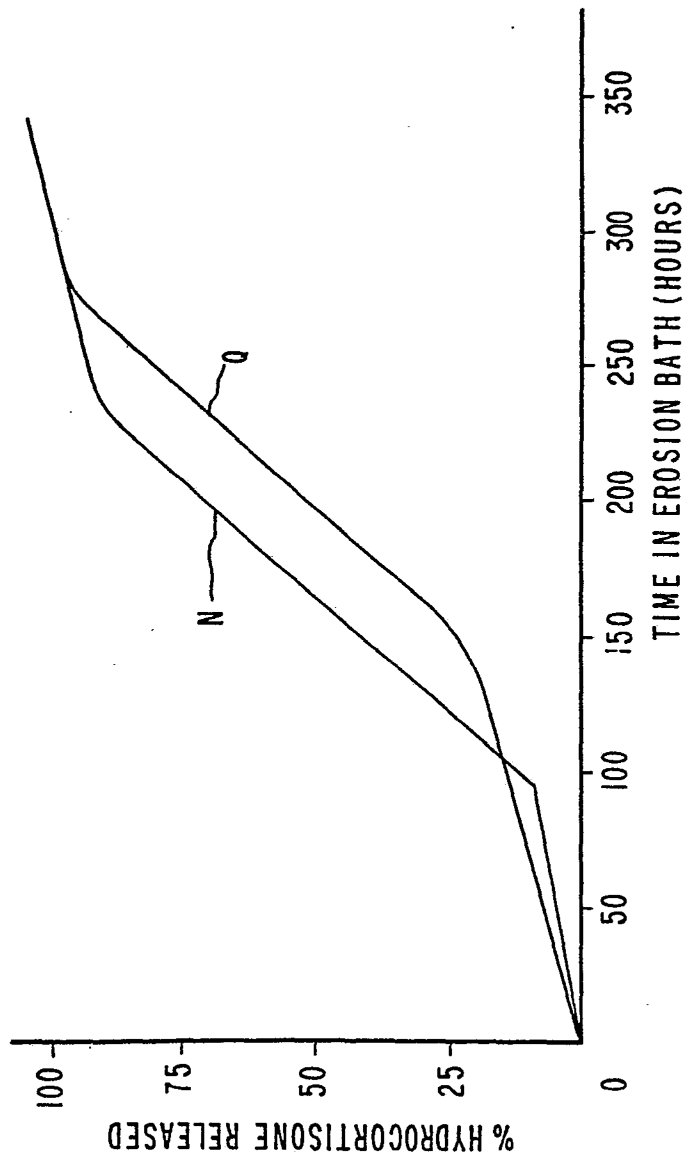
% HYDROCORTISONE RELEASED
0
25
50
75
100
TIME IN EROSION BATH (HOURS)
50
100
150
200
250
300
350
N
Q

FIG. 10

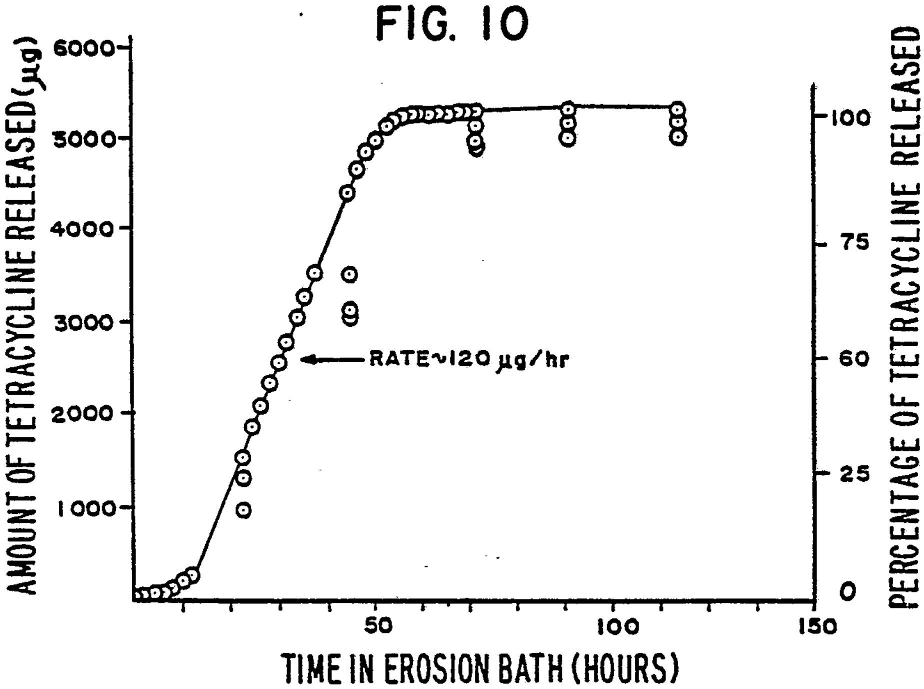

6000
5000
4000
3000
2000
1000
AMOUNT OF TETRACYCLINE RELEASED (μg)
RATE~120 μg/hr
100
75
60
25
0
PERCENTAGE OF TETRACYCLINE RELEASED
50
100
150
TIME IN EROSION BATH (HOURS)


FIG. 11

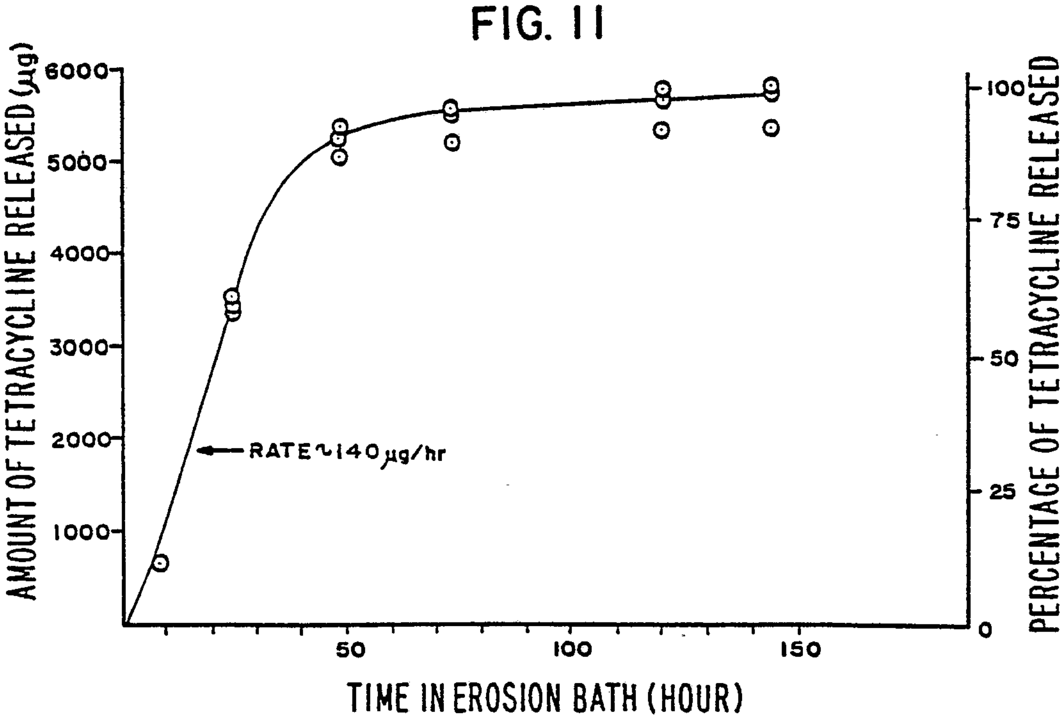

6000
5000
4000
3000
2000
1000
AMOUNT OF TETRACYCLINE RELEASED (μg)
RATE~140 μg/hr
100
75
50
25
0
PERCENTAGE OF TETRACYCLINE RELEASED
50
100
150
TIME IN EROSION BATH (HOUR)

FIG. 12

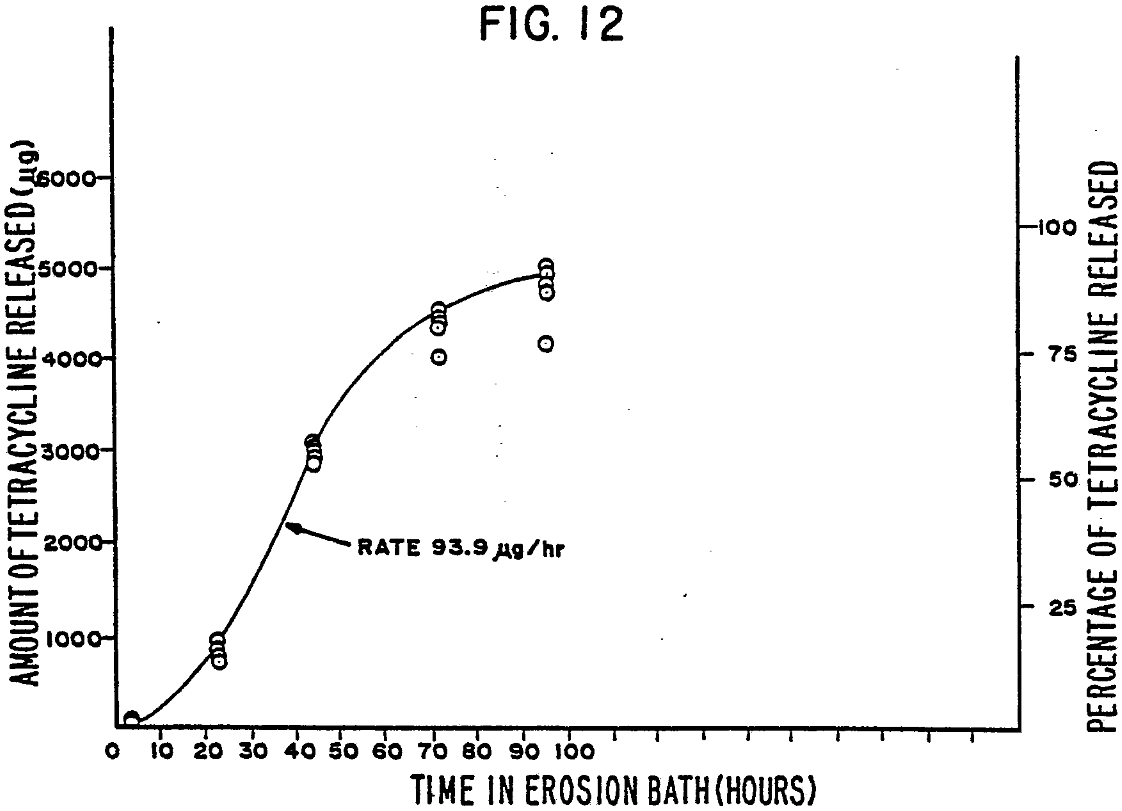
AMOUNT OF TETRACYCLINE RELEASED (µg)
6000
5000
4000
3000
2000
1000
PERCENTAGE OF TETRACYCLINE RELEASED
100
75
50
25
RATE 93.9 µg/hr
0 10 20 30 40 50 60 70 80 90 100
TIME IN EROSION BATH (HOURS)

FIG. 13

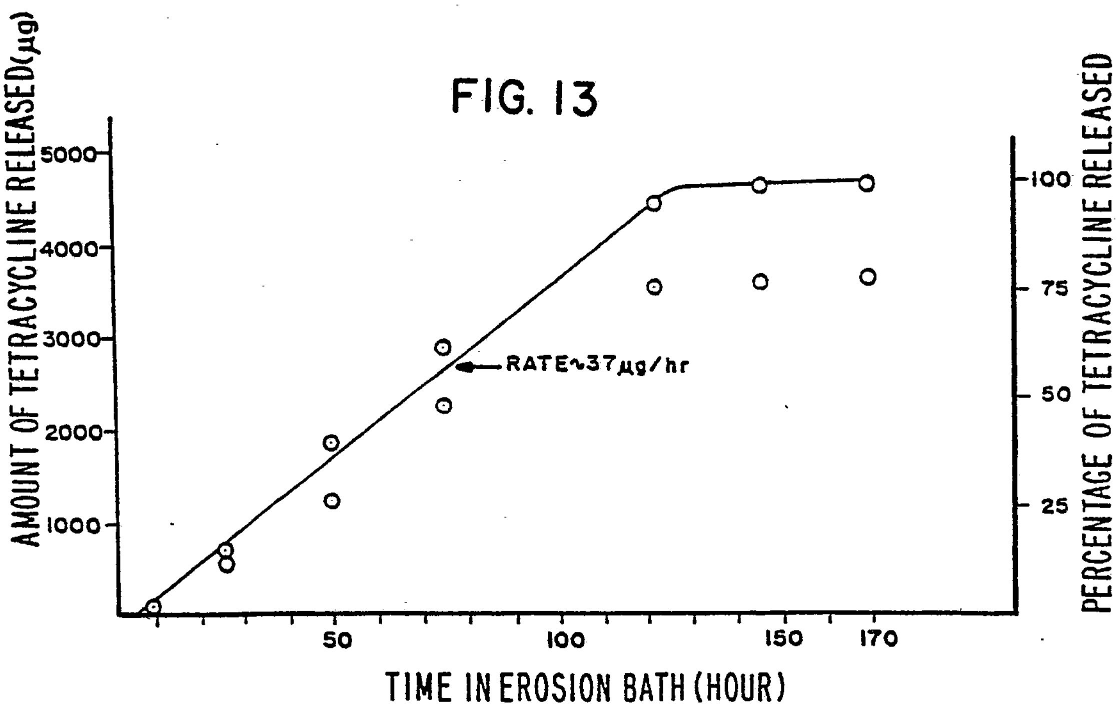
AMOUNT OF TETRACYCLINE RELEASED (µg)
5000
4000
3000
2000
1000
PERCENTAGE OF TETRACYCLINE RELEASED
100
75
50
25
RATE~37 µg/hr
50
100
150
170
TIME IN EROSION BATH (HOUR)

FIG. 14

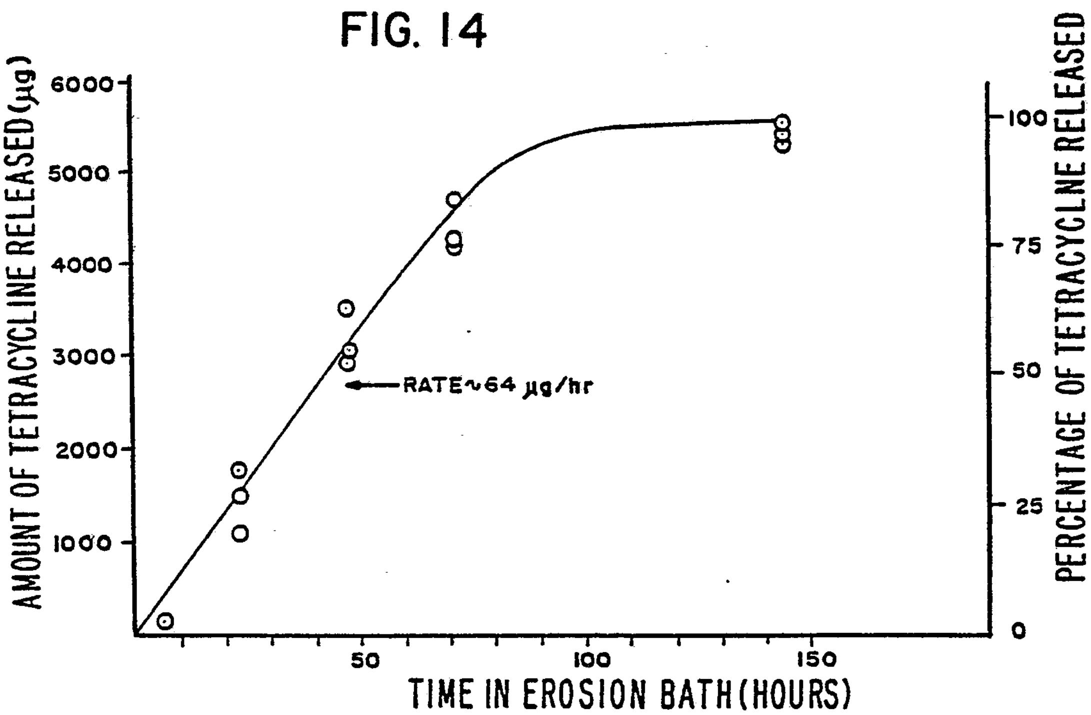

6000
5000
4000
3000
2000
1000
AMOUNT OF TETRACYCLINE RELEASED (μg)
RATE∿64 μg/hr
50
100
150
TIME IN EROSION BATH (HOURS)
100
75
50
25
0
PERCENTAGE OF TETRACYCLINE RELEASED


FIG. 15

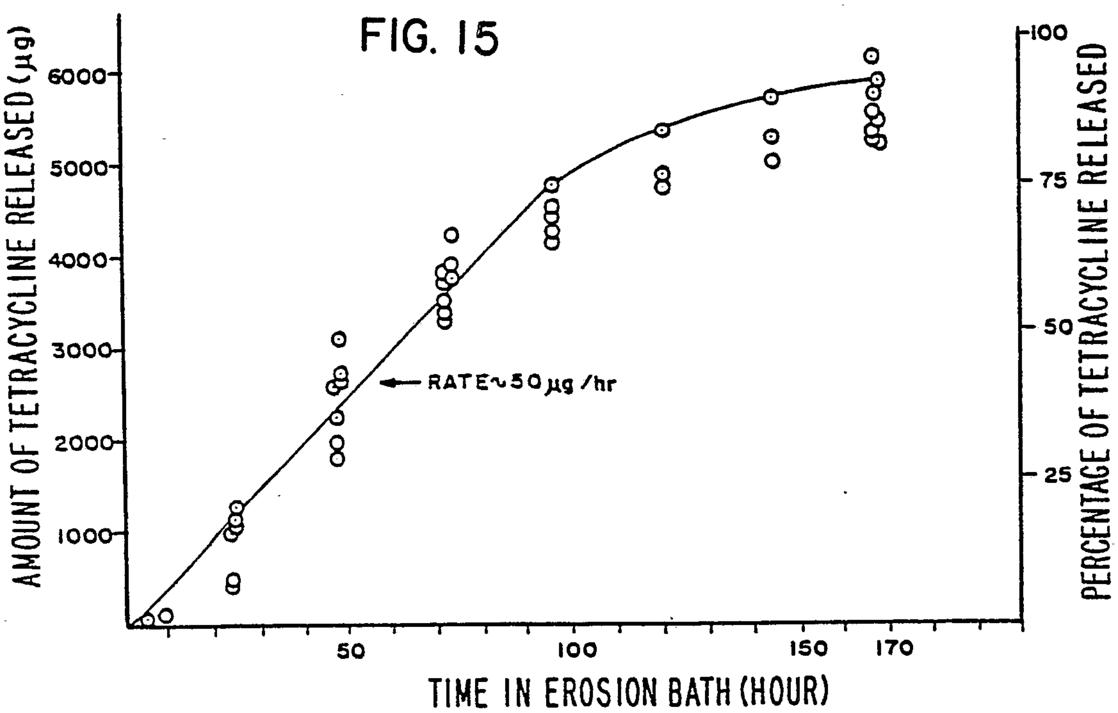

6000
5000
4000
3000
2000
1000
AMOUNT OF TETRACYCLINE RELEASED (μg)
RATE∿50 μg/hr
50
100
150
170
TIME IN EROSION BATH (HOUR)
100
75
50
25
PERCENTAGE OF TETRACYCLINE RELEASED

FIG. 16

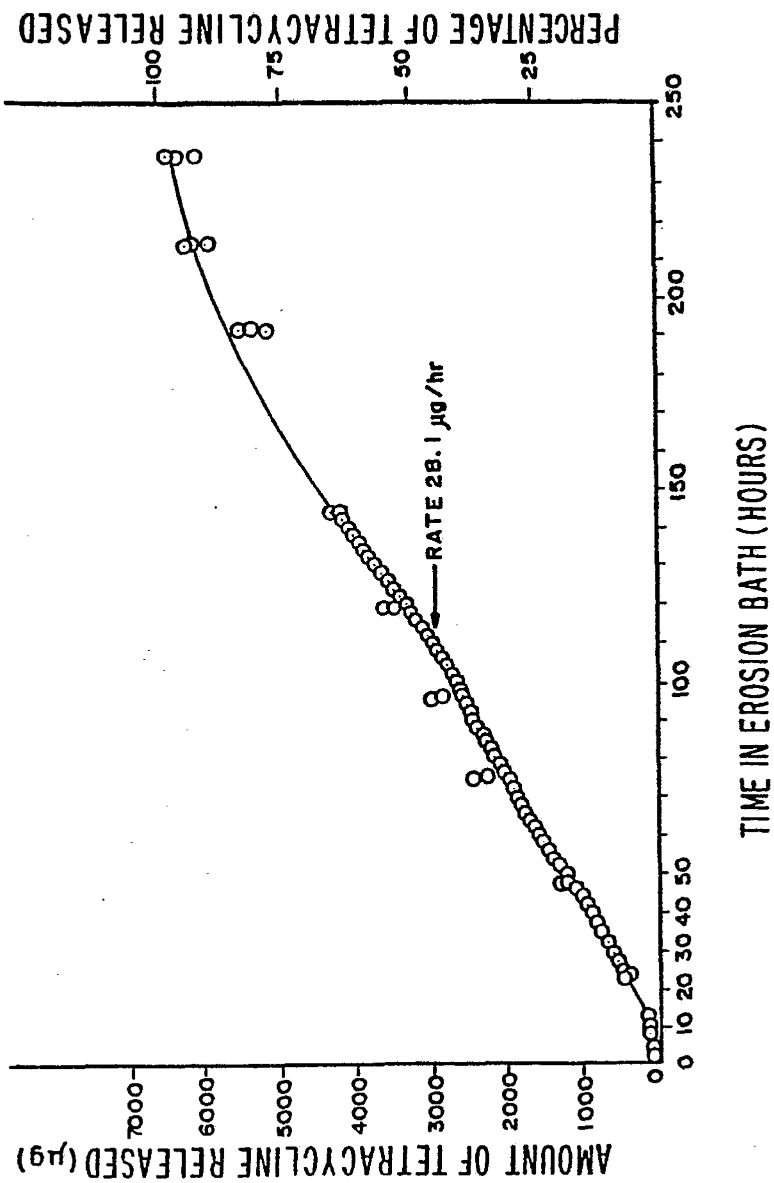

PERCENTAGE OF TETRACYCLINE RELEASED
100
75
50
25
AMOUNT OF TETRACYCLINE RELEASED (μg)
7000
6000
5000
4000
3000
2000
1000
0
RATE 28.1 μg/hr
0
10
20
30
40
50
100
150
200
250
TIME IN EROSION BATH (HOURS)